# EUROPÄISCHE PATENTSCHRIFT

(11) **EP 0 657 432 B1**
(45) Veröffentlichungstag und Bekanntmachung des Hinweises auf die Patenterteilung: **12.03.2003**
(21) Anmeldenummer: 94118677.7
(22) Anmeldetag: 28.11.1994
(51) Int. Cl.: C07D 211/90, A61K 31/44

(54) **Phenyl-substituierte 1,4-Dihydropyridine mit cerebraler Aktivität**
Phenyl-substituted 1,4-dihydropyridines with cerebral activity
1,4-Dihydropyridines substitués par un groupement phényle avec une activité cérébrale

(30) Priorität: 10.12.1993 DE 4342194; 10.12.1993 DE 4342196
(43) Veröffentlichungstag der Anmeldung: 14.06.1995
(73) Patentinhaber: BAYER AG, 51368 Leverkusen (DE)
(72) Erfinder: Meier, Heinrich, Dr., Kobe 658 (JP); Hartwig, Wolfgang, Dr., Stamford, Connecticut 06903 (US); Junge, Bodo, Dr., D-42399 Wuppertal (DE); Schohe-Loop, Rudolf, Dr., D-42327 Wuppertal (DE); Gao, Zhan, Dr., Beijing 100016 (CN); Schmidt, Bernard, Dr., D-51789 Lindlar (DE); de Jonge, Maarten, Dr., D-51491 Overath (DE); Schuurman, Teunis Dr., D-53797 Lohmar (DE)

(56) Entgegenhaltungen:
- EP-A- 0 007 293
- EP-A- 0 088 940
- EP-A- 0 525 568
- EP-A- 0 534 520
- EP-A- 0 595 164
- DE-A- 2 117 572
- DE-A- 2 117 573
- US-A- 3 932 646
- US-A- 3 966 946
- JOURNAL OF CARDIOVASCULAR PHARMACOLOGY; SUPPLEMENT 1, Bd.10, 1987, NEW YORK Seiten S60 - S65 PEDER BERNTSSON ET AL. 'Felodipine Analogs: Structure - Activity Relationships'

## Beschreibung

Die Erfindung betrifft neue Phenyl-substituierte 1,4-Dihydropyridine, Verfahren zu ihrer Herstellung und ihre Verwendung in Arzneimitteln, insbesondere als cerebral wirksame Mittel.

Es ist bekannt, daß einige Dihydropyridine, wie z.B. Nimodipin eine cerebrale Wirksamkeit besitzen [vgl. DOS 28 15 578]. Es sind auch kreislaufwirksame Dihydropyridine bekannt, die in 4-Position einen Phenylring tragen, der durch Halogen, CN oder CF₃ substituiert ist [vgl. DOS 1 963 188, DOS 2 117 572, DOS 2 117 573 und EP 007 293).

Substituierte 1,4-Dihydropyridine sind als Synthesezwischenstufen bekannt [EP-A-0 595 164 (nachveröffentlicht), EP-A-0 088 940, EP-A- 0 525 568].

Die EP-A-0 534 520 beschreibt Verfahren zur Herstellung von substituierten 1,4-Dihydropyridinen.

Weiterhin ist die antihypertensive und vasospasmolytische Wirkung von substituierten 1,4-Dihydropyridinen bekannt (US 3 932 646, J. Cardiovasc. Pharmacol. 1987, 10, S60-S65, US 3 966 946, DE-A- 25 08 181).

Die EP-A-0 525 568 offenbart außerdem N-alkylierte 1,4-Dihydropyridine als Cerebraltherapeutika.

Die vorliegende Erfindung betrifft die Verwendung von 4-Phenyl-substituierten 1,4-Dihydropyridinen der allgemeinen Formel (I) 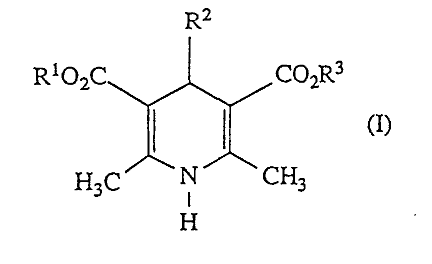 in welcher,
- R¹ und R³: gleich oder verschieden sind und für geradkettiges oder verzweigtes Alkyl mit bis zu 8 Kohlenst6ffatomen stehen, das gegebenenfalls durch geradkettiges oder verzweigtes Alkoxy mit bis zu 6 Kohlenstoffatomen oder Hydroxy substituiert ist, oder
für Cycloalkyl mit 3 bis 7 Kohlenstoffatomen stehen,
und R² für den Rest 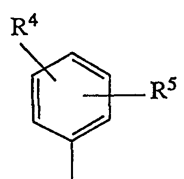 steht,
worin
- R⁴ und R⁵: gleich oder verschieden sind und für Halogen, Cyano, Ethinyl, Trifluormethoxy, Methyl, Methylthio, Trifluormethyl oder für geradkettiges oder verzweigtes Alkoxy mit bis zu 4 Kohlenstoffatomen stehen,
oder
R⁴ oder R⁵ für Wasserstoff steht
und deren Salze, insbesondere die unter die Formel (I) fallenden neuen Verbindungen der Ausführungsbeispiele 1-124, zur Herstellung eines Arzneimittels zur Behandlung von zentral degenerativen Erkrankungen oder Hirnleistungsstörungen.

Physiologisch unbedenkliche Salze sind Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt Verbindung Bevorzugt ist die erfindungsgem dungen der allgemeinen Formel (I)
in welcher,
- R¹ und R³: gleich oder verschieden sind und für geradkettiges oder verzweigtes Alkyl mit bis zu 8 Kohlenstoffatomen stehen, das gegebenenfalls durch geradkettiges oder verzweigtes Alkoxy mit bis zu 5 Kohlenstoffatomen oder Hydroxy substituiert ist, oder
für Cyclopropyl, Cyclopentyl, Cyclohexyl oder Cycloheptyl stehen,
und R² für den Rest 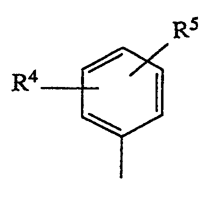 steht,
worin
- R⁴ und R⁵: gleich oder verschieden sind und für Fluor, Brom, Chlor, Cyano, Ethinyl, Trifluormethoxy, Methyl, Methylthio, Trifluormethyl oder für geradkettiges oder verzweigtes Alkoxy mit bis zu 3 Kohlenstoffatomen stehen, oder
R⁴ oder R⁵ für Wasserstoff steht
und deren Salze.

Besonders hervorzuheben ist die erfindungsgemäße Verwendung von Verbindungen der allgemeinen Formel I, in welcher R³ für den Rest -(CH₂)ₙ-OR⁶ steht, worin n für eine Zahl von 2 bis 4 steht und R⁶ für Wasserstoff oder Alkyl mit 1 bis 4 C-Atomen steht, insbesondere Verbindungen der Formel I in welcher R³ für den Rest -CH₂-CH₂-OCH₃ steht, und R¹ mit R³ gleich oder verschieden ist und für geradkettiges oder verzweigtes Alkyl mit bis zu 8 Kohlenstoffatomen steht, welches gegebenenfalls durch Hydroxy oder Alkoxy mit 1 bis 4 C-Atomen substituiert ist, oder für Cyclopropyl, Cyclopentyl, Cyclohexyl oder Cycloheptyl steht

Von besonderem Interesse ist die erfindungsgemäße Verwendung von Verbindungen der allgemeinen Formel I, in welcher R² für einen Cyanophenylrest steht, der als zweiten Phenylsubstituenten Fluor, Chlor oder CF₃ trägt. Von besonderem Interesse sind auch solche Verbindungen der allgemeinen Formel I, die in 2- und 3-Position des Phenylrestes R² durch Substituenten aus der Gruppe Chlor, Fluor, Cyano oder CF₃ substituiert sind, wobei der 2,3-Dichlorphenylrest ausgeschlossen wird.

Außerdem betrifft die Erfindung folgende Verbindungen der allgemeinen Formel (I):
(±) Isopropyl-(2-methoxyethyl)-4-(2-fluor-phenyl)-1,4-dihydro-2,6-dimethylpyridin-3,5-dicarboxylat
(+) Isopropyl-(2-methoxyethyl)-4-(2-fluor-phenyl)-1,4-dihydro-2,6-dimethylpyridin-3,5-dicarboxylat
(-) Isopropyl-(2-methoxyethyl)-4-(2-fluor-phenyl)-1,4-dihydro-2,6-dimethylpyridin-3,5-dicarboxylat
(±) Isopropyl-(2-methoxyethyl)-4-(2,3-difluor-phenyl)-1,4-dihydro-2,6-dimethylpyridin-3,5-dicarboxylat
(+) Isopropyl-(2-methoxyethyl)-4-(2,3-difluor-phenyl)-1,4-dihydro-2,6-dimethylpyridin-3,5-dicarboxylat
(-) Isopropyl-(2-methoxyethyl)-4-(2,3-difluor-phenyl)-1,4-dihydro-2,6-dimethylpyridin-3,5-dicarboxylat
(±) Isopropyl-(2-methoxyethyl)-4-(2,5-difluor-phenyl)-1,4-dihydro-2,6-dimethylpyridin-3,5-dicarboxylat
(+) Isopropyl-(2-methoxyethyl)-4-(2,5-difluor-phenyl)-1,4-dihydro-2,6-dimethylpyridin-3,5-dicarboxylat
(-) Isopropyl-(2-methoxyethyl)-4-(2,5-difluor-phenyl)-1,4-dihydro-2,6-dimethylpyridin-3,5-dicarboxylat
(±) Isopropyl-(2-methoxyethyl)-4-(2,6-difluor-phenyl)-1,4-dihydro-2,6-dimethylpyridin-3,5-dicarboxylat
(+) Isopropyl-(2-methoxyethyl)-4-(2,6-difluor-phenyl)-1,4-dihydro-2,6-dimethylpyridin-3,5-dicarboxylat
(-) Isopropyl-(2-methoxyethyl)-4-(2,6-difluor-phenyl)-1,4-dihydro-2,6-dimethylpyridin-3,5-dicarboxylat
(±) Isopropyl-(2-methoxyethyl)-4-(2,5-dichlor-phenyl)-1,4-dihydro-2,6-dimethylpyridin-3,5-dicarboxylat
(+) Isopropyl-(2-methoxyethyl)-4-(2,5-dichlor-phenyl)-1,4-dihydro-2,6-dimethylpyridin-3,5-dicarboxylat
(-) Isopropyl-(2-methoxyethyl)-4-(2,5-dichlor-phenyl)-1,4-dihydro-2,6-dimethylpyridin-3,5-dicarboxylat
(±) Isopropyl-(2-methoxyethyl)-4-(2-chlor-6-fluor-phenyl)-1,4-dihydro-2,6-dimethylpyridin-3,5-dicarboxylat
(+) Isopropyl-(2-methoxyethyl)-4-(2-chlor-6-fluor-phenyl)-1,4-dihydro-2,6-dimethylpyridin-3,5-dicarboxylat
(-) Isopropyl-(2-methoxyethyl)-4-(2-chlor-6-fluor-phenyl)-1,4-dihydro-2,6-dimethylpyridin-3,5-dicarboxylat
(±) Isopropyl-(2-methoxyethyl)-4-(2-fluor-3-trifluor-methylphenyl)-1,4-dihydro-2,6-dimethylpyridin-3,5-dicarboxylat
(+) Isopropyl-(2-methoxyethyl)-4-(2-fluor-3-trifluor-methylphenyl)-1,4-dihydro-2,6-dimethylpyridin-3,5-dicarboxylat
(-) Isopropyl-(2-methoxyethyl)-4-(2-fluor-3-trifluor-methylphenyl)-1,4-dihydro-2,6-dimethylpyridin-3,5-dicarboxylat
(±) Isopropyl-(2-methoxyethyl)-4-(3-chlor-2-cyano-phenyl)-1,4-dihydro-2,6-dimethylpyridin-3,5-dicarboxylat
(+) Isopropyl-(2-methoxyethyl)-4-(3-chlor-2-cyano-phenyl)-1,4-dihydro-2,6-dimethylpyridin-3,5-dicarboxylat
(-) Isopropyl-(2-methoxyethyl)-4-(3-chlor-2-cyano-phenyl)-1,4-dihydro-2,6-dimethylpyridin-3,5-dicarboxylat
(±) Isopropyl-(2-methoxyethyl)-4-(2-cyano-phenyl)-1,4-dihydro-2,6-dimethylpyridin-3,5-dicarboxylat
(+) Isopropyl-(2-methoxyethyl)-4-(2-cyano-phenyl)-1,4-dihydro-2,6-dimethylpyridin-3,5-dicarboxylat
(-) Isopropyl-(2-methoxyethyl)-4-(2-cyano-phenyl)-1,4-dihydro-2,6-dimethylpyridin-3,5-dicarboxylat
(±) tert.Butyl-(2-methoxyethyl)-4-(3-cyano-phenyl)-1,4-dihydro-2,6-dimethylpyridin-3,5-dicarboxylat
(+) tert.Butyl-(2-methoxyethyl)-4-(3-cyano-phenyl)-1,4-dihydro-2,6-dimethylpyridin-3,5-dicarboxylat
(-) tert.Butyl-(2-methoxyethyl)-4-(3-cyano-phenyl)-1,4-dihydro-2,6-dimethylpyridin-3,5-dicarboxylat
(±) Cycloheptyl-(2-methoxyethyl)-4-(3-cyano-phenyl)-1,4-dihydro-2,6-dimethylpyridin-3,5-dicarboxylat
(+) Cycloheptyl-(2-methoxyethyl)-4-(3-cyano-phenyl)-1,4-dihydro-2,6-dimethylpyridin-3,5-dicarboxylat
(-) Cycloheptyl-(2-methoxyethyl)-4-(3-cyano-phenyl)-1,4-dihydro-2,6-dimethylpyridin-3,5-dicarboxylat
(±) Cyclopentyl-(2-methoxyethyl)-4-(2-chlor-3-cyano-phenyl)-1,4-dihydro-2,6-dimethylpyridin-3,5-dicarboxylat
(+) Cyclopentyl-(2-methoxyethyl)-4-(2-chlor-3-cyano-phenyl)-1,4-dihydro-2,6-dimethylpyridin-3,5-dicarboxylat
(-) Cyclopentyl-(2-methoxyethyl)-4-(2-chlor-3-cyano-phenyl)-1,4-dihydro-2,6-dimethylpyridin-3,5-dicarboxylat
(±) Cyclopentyl-(2-methoxyethyl)-4-(2-fluor-3-trifluormethyl-phenyl)-1,4-dihydro-2,6-dimethylpyridin-3,5-dicarboxylat
(+) Cyclopentyl-(2-methoxyethyl)-4-(2-fluor-3-trifluormethyl-phenyl)-1,4-dihydro-2,6-dimethylpyridin-3,5-dicarboxylat
(-) Cyclopentyl-(2-methoxyethyl)-4-(2-fluor-3-trifluormethyl-phenyl)-1,4-dihydro-2,6-dimethylpyridin-3,5-dicarboxylat
(±) (2-Methoxyethyl)-(methyl)-4-(2-fluor-3-trifluormethyl-phenyl)-1,4-dihydro-2,6-dimethylpyridin-3,5-dicarboxylat
(+) (2-Methoxyethyl)-(methyl)-4-(2-fluor-3-trifluormethyl-phenyl)-1,4-dihydro-2,6-dimethylpyridin-3,5-dicarboxylat
(-) (2-Methoxyethyl)-(methyl)-4-(2-fluor-3-trifluormethyl-phenyl)-1,4-dihydro-2,6-dimethylpyridin-3,5-dicarboxylat
(±) Cyclopentyl-(2-methoxyethyl)-4-(2-cyano-3-chlor-phenyl)-1,4-dihydro-2,6-dimethylpyridin-3,5-dicarboxylat
(+) Cyclopentyl-(2-methoxyethyl)-4-(2-cyano-3-chlor-phenyl)-1,4-dihydro-2,6-dimethylpyridin-3,5-dicarboxylat
(-) Cyclopentyl-(2-methoxyethyl)-4-(2-cyano-3-chlor-phenyl)-1,4-dihydro-2,6-dimethylpyridin-3,5-dicarboxylat

Die Erfindung betrifft auch Verfahren zur Herstellung der erfindungsgemäßen Verbindungen der allgemeinen Formel (I),
in welcher
R² für den Rest 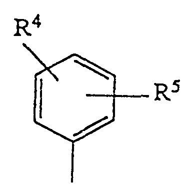 steht,
ausgewählt aus einer Gruppe von Verbindungen, in der die Substituenten R¹, R³, R⁴ und R⁵ die in der folgenden Tabelle aufgeführten Tabelle aufgeführten Bedeutungen haben:

| **R**^{**1**} | **R**^{**3**} | **R**^{**4**} | **R**^{**5**} | **Racemat, (+)-, (-)-Enantiomer** |
|---|---|---|---|---|
| isopropyl | CH₂CH₂OCH₃ | 2-F | H | Racemat ; |
| cycloheptyl | CH₂CH₂OCH₃ | 3-CN | H | (-) ; |
| isopropyl | CH₂CH₂OCH₃ | 2-Cl | 6-F | Racemat ; |
| isopropyl | CH₂CH₂OCH₃ | 2-Cl | 6-F | (+) ; |
| isopropyl | CH₂CH₂OCH₃ | 2-Cl | 6-F | (-) ; |
| isopropyl | CH₂CH₂OCH₃ | 2-F | 6-F | Racemat ; |
| isopropyl | CH₂CH₂OCH₃ | 2-F | 6-F | (+) ; |
| isopropyl | CH₂CH₂OCH₃ | 2-F | 6-F | (-) ; |
| isopropyl | CH₂CH₂OCH₃ | 2-F | 3-F | Racemat ; |
| isopropyl | CH₂CH₂OCH₃ | 2-F | 3-F | (+) ; |
| isopropyl | CH₂CH₂OCH₃ | 2-F | 3-F | (-) ; |
| isopropyl | CH₂CH₂OCH₃ | 2-Cl | 5-Cl | Racemat ; |
| isopropyl | CH₂CH₂OCH₃ | 2-Cl | 5-Cl | (+) ; |
| isopropyl | CH₂CH₂OCH₃ | 2-Cl | 5-Cl | (-) ; |
| isopropyl | CH₂CH₂OCH₃ | 2-CN | 3-Cl | Racemat ; |
| isopropyl | CH₂CH₂OCH₃ | 2-CN | 3-Cl | (+) ; |
| isopropyl | CH₂CH₂OCH₃ | 2-CN | 3-Cl | (-) ; |
| isopropyl | CH₂CH₂OCH₃ | 3-F | 4-F | Racemat ; |
| isopropyl | CH₂CH₂OCH₃ | 3-F | 4-F | (+) ; |
| isopropyl | CH₂CH₂OCH₃ | 3-F | 4-F | (-) ; |
| isopropyl | CH₂CH₂OCH₃ | 2-F | 5-F | Racemat ; |
| isopropyl | CH₂CH₂OCH₃ | 2-F | 5-F | (+) ; |
| isopropyl | CH₂CH₂OCH₃ | 2-F | 5-F | (-) ; |
| isopropyl | CH₂CH₂OCH₃ | 2-CN | H | Racemat ; |
| isopropyl | CH₂CH₂OCH₃ | 2-CN | H | (+) ; |

| R¹ | R³ | R⁴ | R⁵ | Racemat, (+)-, (-)-Enantiomer |
|---|---|---|---|---|
| isopropyl | CH₂CH₂OCH₃ | 2-CN | H | (-) ; |
| isopropyl | CH₂CH₂OCH₃ | 2-F | 3-CF₃ | Racemat ; |
| isopropyl | CH₂CH₂OCH₃ | 2-F | 3-CF₃ | (+) ; |
| isopropyl | CH₂CH₂OCH₃ | 2-F | 3-CF₃ | (-) ; |
| cyclopentyl | CH₂CH₂OCH₃ | 2-Cl | 3-CN | Racemat ; |
| cyclopentyl | CH₂CH₂OCH₃ | 2-Cl | 3-CN | (+) ; |
| cyclopentyl | CH₂CH₂OCH₃ | 2-Cl | 3-CN | (-) ; |
| t-butyl | CH₂CH₂OCH₃ | 3-CN | H | Racemat ; |
| t-butyl | CH₂CH₂OCH₃ | 3-CN | H | (+) ; |
| t-butyl | CH₂CH₂OCH₃ | 3-CN | H | (-) ; |
| cyclopentyl | CH₂CH₂OCH₃ | 2-F | 3-CF₃ | Racemat ; |
| cyclopentyl | CH₂CH₂OCH₃ | 2-F | 3-CF₃ | (+) ; |
| cyclopentyl | CH₂CH₂OCH₃ | 2-F | 3-CF₃ | (-) ; |
| methyl | CH₂CH₂OCH₃ | 2-F | 3-CF₃ | Racemat ; |
| methyl | CH₂CH₂OCH₃ | 2-F | 3-CF₃ | (+) ; |
| methyl | CH₂CH₂OCH₃ | 2-F | 3-CF₃ | (-) ; |
| cycloheptyl | CH₂CH₂OCH₃ | 3-CN | H | Racemat ; |
| cycloheptyl | CH₂CH₂OCH₃ | 3-CN | H | (+) ; |
| isopropyl | CH₂CH₂OCH₃ | 2-F | H | (+) ; |
| isopropyl | CH₂CH₂OCH₃ | 2-F | H | (-) ; |
| cyclopentyl | CH₂CH₂OCH₃ | 2-CN | 3-Cl | Racemat ; |
| cyclopentyl | CH₂CH₂OCH₃ | 2-CN | 3-Cl | (+) ; |
| cyclopentyl | CH₂CH₂OCH₃ | 2-CN | 3-Cl | (-) ; |
| isopropyl | CH₂CH₂OCH₃ | 2-Cl | 5-CN | Racemat ; |
| cycloheptyl | CH₂CH₂OCH₃ | 3-CN | H | (-) ; |
| isopropyl | CH₂CH₂OCH₃ | 2-F | 4-F | Racemat ; |
| isopropyl | CH₂CH₂OCH₃ | 3-F | H | Racemat ; |
| isopropyl | CH₂CH₂OCH₃ | 2-Cl | 4-Cl | Racemat ; |
| isopropyl | CH₂CH₂OCH₃ | 3-Cl | 4-Cl | Racemat ; |
| isopropyl | CH₂CH₂OCH₃ | 3-Cl | 5-Cl | Racemat ; |
| isopropyl | CH₂CH₂OCH₃ | 2-Cl | 6-Cl | Racemat ; |
| isopropyl | CH₂CH₂OCH₃ | 2-F | 5-Cl | Racemat ; |
| isopropyl | CH₂CH₂OCH₃ | 3-Cl | 4-Cl | Racemat ; |
| isopropyl | CH₂CH₂OCH₃ | 2-CH₃ | 3-F | Racemat ; |
| isopropyl | CH₂CH₂OCH₃ | 2-C=CH | 3-Cl | Racemat ; |
| isopropyl | CH₂CH₂OCH₃ | 2-Cl | 5-C=CH | Racemat ; |
| isopropyl | CH₂CH₂OCH₃ | 2-C=CH | 4-F | Racemat ; |
| isopropyl | CH₂CH₂OCH₃ | 3-OCF₃ | H | Racemat ; |
| isopropyl | CH₂CH₂OCH₃ | 2-SCH₃ | 5-Br | Racemat ; |
| isopropyl | CH₂CH₂OCH₃ | 2-OCH₃ | 3-F | Racemat ; |
| isopropyl | CH₂CH₂OCH₃ | 2-OCH₃ | 6-F | Racemat ; |
| isopropyl | CH₂CH₂OCH₃ | 2-OCH₃ | 5-F | Racemat ; |
| isopropyl | CH₂CH₂OCH₃ | 2-F | 5-OCH₃ | Racemat ; |
| isopropyl | CH₂CH₂OCH₃ | 3-Cl | H | Racemat ; |
| isopropyl | CH₂CH₂OCH₃ | 2-OCH₃ | 6-Cl | Racemat ; |
| isopropyl | CH₂CH₂OCH₃ | 2-Cl | 5-CN | Racemat ; |
| isopropyl | CH₂CH₂OCH₃ | 2-OCH₃ | 5-Cl | Racemat ; |
| isopropyl | CH₂CH₂OCH₃ | 2-F | 5-F | Racemat ; |
| isobutyl | CH₂CH₂OCH₃ | 3-F | 4-F | Racemat ; |
| 3-butyl | CH₂CH₂OCH₃ | 3-F | 4-F | Racemat ; |
| cyclopentyl | CH₂CH₂OCH₃ | 3-F | 4-F | Racemat ; |
| cyclohexyl | CH₂CH₂OCH₃ | 3-F | 4-F | Racemat ; |
| isopropyl | n-butyl | 2-F | 6-Cl | Racemat ; |
| cyclopentyl | n-butyl | 2-F | 6-Cl | Racemat ; |
| methyl | CH₂CH₂OCH₃ | 3-CN | H | Racemat ; |
| isopropyl | CH₂CH₂OH | 3-CN | H | Racemat ; |
| isopropyl | CH₂CH₂OCH₂CH₃ | 3-CN | H | Racemat ; |
| isopropyl | CH₂CH₂OCH(CH₃)₂ | 3-CN | H | Racemat ; |
| isopropyl | CH₂CH₂OCH₂CH(CH₃)₂ | 3-CN | H | Racemat ; |
| isopropyl | CH₂CH₂OC(CH₃)₃ | 3-CN | H | Racemat ; |
| isopropyl | CH₂CH₂CH₂OCH₃ | 3-CN | H | Racemat ; |
| isopropyl | CH₂C(CH₃)₂CH₂OCH₃ | 3-CN | H | Racemat ; |
| isobutyl | CH₂CH₂OCH₃ | 3-CN | H | Racemat ; |
| cyclopentyl | CH₂CH₂OH | 3-CN | H | Racemat ; |
| cyclopentyl | CH₂CH₂OCH₃ | 3-CN | H | Racemat ; |
| cyclopentyl | CH₂CH₂OCH₂CH₃ | 3-CN | H | Racemat ; |
| cyclopentyl | CH₂CH₂OCH(CH₃)₂ | 3-CN | H | Racemat ; |
| cyclopentyl | CH₂CH₂OCH(CH₃)₂ | 3-CN | H | Racemat ; |
| cyclopentyl | CH₂CH₂OC(CH₃)₃ | 3-CN | H | Racemat ; |
| cyclopentyl | CH₂C(CH₃)₂CH₂OCH₃ | 3-CN | H | Racemat ; |
| cyclohexyl | CH₂CH₂OCH₃ | 3-CN | H | Racemat ; |
| CH₂CH₂OCH₃ | CH₂CH₂OCH₃ | 3-CN | H | ; |
| cyclopentyl | CH₂CH₂OCH₃ | 2-Cl | 5-CN | Racemat ; |
| cyclopentyl | methyl | 2-CN | 3-Cl | Racemat ; |
| t-butyl | CH₂CH₂OCH₃ | 2-F | 6-F | Racemat ; |
| cyclopentyl | CH₂CH₂OCH(CH₃)₂ | 2-F | 3-F | Racemat ; |
| cyclopentyl | CH₂CH₂OH | 2-F | 3-F | Racemat ; |
| isopropyl | CH₂CH₂OH | 2-F | 3-F | Racemat ; |
| isopropyl | CH₂CH₂OCH(CH₃)₂ | 2-F | 3-F | Racemat ; |
| cycloheptyl | CH₂CH₂OCH₃ | 2-F | 5-F | Racemat ; |
| isopropyl | CH₂C(CH₃)₂OH | 2-F | 5-F | Racemat ; |
| Methyl | CH₂CH₂OCH₃ | 3-CN | H(-) | ; |
| methyl | CH₂CH₂OCH₃ | 3-CN | H | (+) ; |
| | | | | |
| isopropyl | CH₂CH₂OCH₃ | 2-Cl | 5-CN | (+) ; |
| isopropyl | CH₂CH₂OCH₃ | 2-Cl | 5-CN | (-) ; |
| CH₂CH₂OCH₃ | CH₂CH₂OCH₃ | 2-F | 3-CF₃ | ; |
| CH₂CH₂OCH₃ | CH₂CH₂OCH₃ | 2-Cl | 5-Cl | ; |
| CH₂CH₂OCH₃ | CH₂CH₂OCH₃ | 2-F | 5-F | ; |
| CH₂CH₂CH₂OCH₃ | CH₂CH₂CH₂OCH₃ | 2-Cl | 5-Cl | ; |
| CH₂CH₂CH₂OCH₃ | CH₂CH₂CH₂OCH₃ | 2-F | 5-F | ; |
| CH₂C(CH₃)₂CH₂OCH₃ | CH₂C(CH₃)₂CH₂OCH₃ | 2-Cl | 5-Cl | ; |
| CH₂C(CH₃)₂CH₂OCH₃ | CH₂C(CH₃)₂CH₂OCH₃ | 2-F | 5-F | ; |
| methyl | CH₂CH₂OCH₃ | 2-F | 3-F | Racemat ; |
| CH₂CH₂OCH₃ | CH₂CH₂OCH₃ | 2-F | 3-F | ; |
| CH₂CH₂OCH₃ | CH₂CH₂OCH₃ | 2-Cl | H | ; |
| isopropyl | CH₂CH₂OCH₃ | 2-Cl | H | Racemat ; |
| | | | | oder |
| isopropyl | CH₂C(CH₃)₂CH₂OH | 2-Cl | H | Racemat |

und deren physiologisch unbedenklichen Salze.
dadurch gekennzeichnet, daß man
[A] Aldehyde der allgemeinen Formel (II)

   R²-CHO (II)

   in welcher
   R² die angegebene Bedeutung hat,
   zunächst mit Acetessigestern der allgemeinen Formel (III)

   H₃C-CO-CH₂-CO₂R¹ (III)

   in welcher
   R¹ die angegebene Bedeutung hat,
   gegebenenfalls unter Isolierung der entsprechenden Ylidenverbindungen der
   allgemeinen Formel (IV) 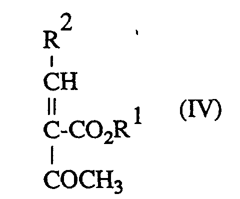 in welcher
   R¹und R² die angegebene Bedeutung haben,
   umsetzt und anschließend entweder mit Verbindungen der allgemeinen Formel (V)

   CH₃-CO-CH₂-CO₂R³ (V)

   in welcher
   - R³: die oben angegebene Bedeutung hat,
   in inerten Lösemitteln, in Anwesenheit von Ammoniak oder Ammoniumsalzen,
   oder direkt mit Enaminoderivaten der allgemeinen Formel (VI) 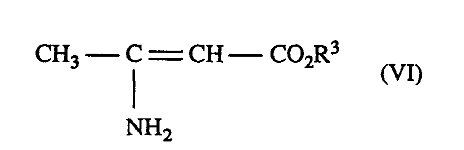 in welcher
   - R³: die angegebene Bedeutung hat,
   umsetzt,
   oder
[B] die Aldehyde der allgemeinen Formel (II) zunächst mit den Verbindungen der allgemeinen Formel (V), gegebenenfalls unter Isolierung der Ylidenverbindungen der allgemeinen Formel (VII) 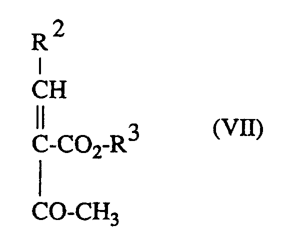 in welcher
   R² und R³ die angegebene Bedeutung haben,
   umsetzt und in einem nächsten Schritt mit den Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII) 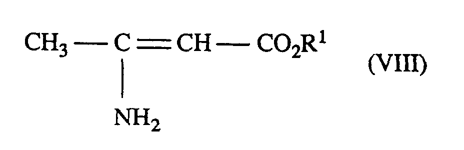 in welcher
   R¹ die angegebene Bedeutung hat,
   umsetzt,
   oder
[C] Verbindungen der allgemeinen Formel (IX) 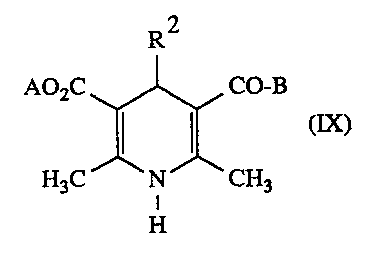
in welcher
R² die oben angegebene Bedeutung hat,
- A: die oben angegebene Bedeutung von R¹ oder R³ hat
und
- B: zusammen mit der -CO-Gruppe eine reaktives Carbonsäurederivat bildet,
in inerten Lösemitteln, in Anwesenheit einer Base,
mit Verbindungen der allgemeinen Formel (X)

R⁶-OH (X)

in welcher
- R⁶: die angegebene Bedeutung von R¹ oder R³ hat,
umsetzt,
und im Fall der reinen Ester-Enantiomere, die enantiomerenreinen Carbonsäuren, gegebenenfalls zunächst über die Stufe eines reaktiven Säurederivates, mit den entsprechenden Alkoholen umsetzt.

Das erfindungsgemäße Verfahren kann durch folgendes Formelschema beispielhaft erläutert werden:

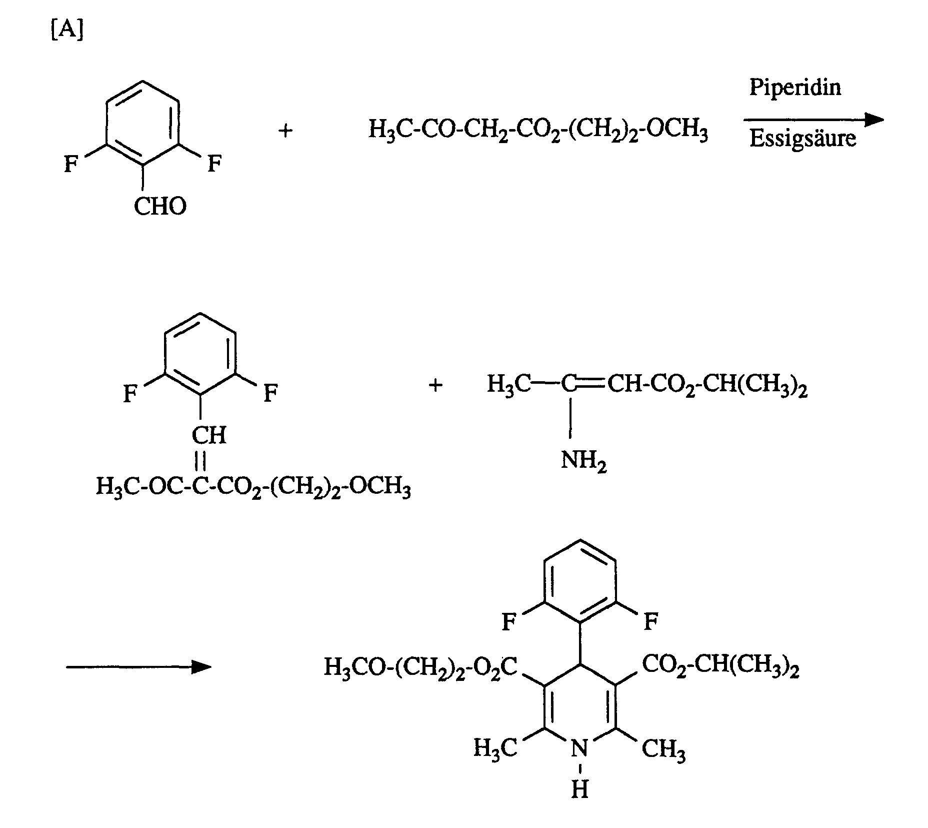

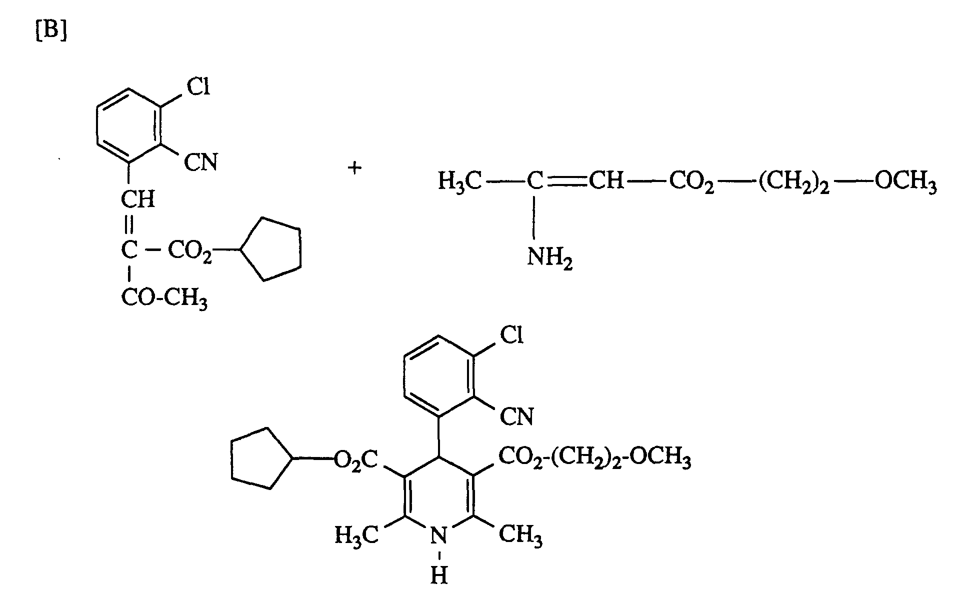

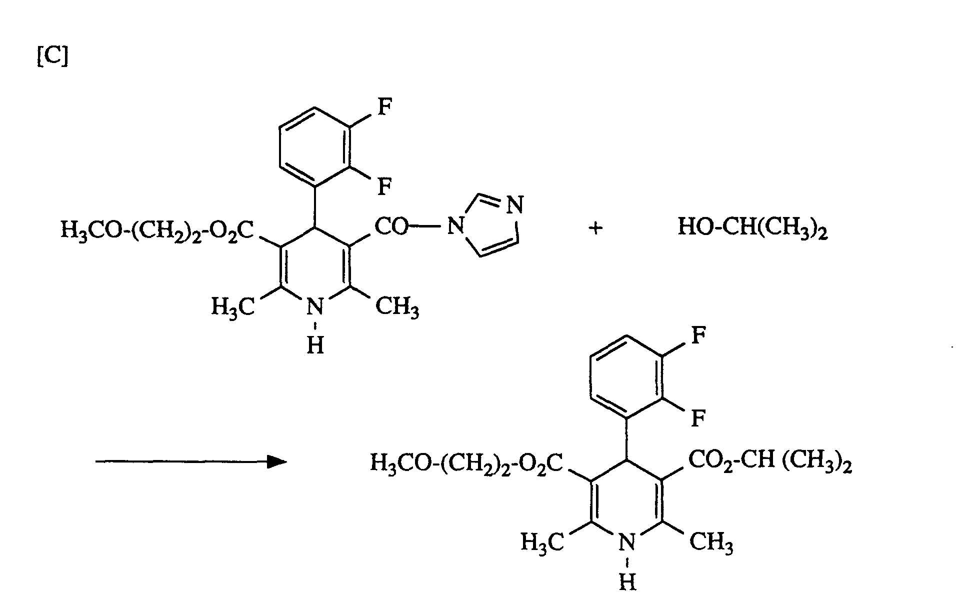

Als Lösemittel für die Verfahren [A] und [B] eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind Isopropanol, Tetrahydrofuran, Methanol, Dioxan und Dimethylformamid.

Als Lösemittel für das Verfahren [C] eignen sich die oben aufgeführten Lösemittel mit Ausnahme der Alkohole und Essigsäure.

Als Basen eignen sich im allgemeinen cyclische Amine, wie beispielsweise Piperidin, C₁-C₃-Tri- und Dialkylamine, wie beispielsweise Di- und Triethylamin oder Pyridin oder Dimethylaminopyridin. Bevorzugt sind in Abhängigkeit der jeweiligen Reaktionsschritte Piperidin, Dimethylaminopyridin und Pyridin.

Als Hilfsstoffe werden bevorzugt Kondensationsmittel eingesetzt die auch Basen sein können. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphonat. Bevorzugt sind N,N'-Dicyclohexylcarbodiimid und Carbonyldiimidazol.

Als Basen eignen sich für die Aktivierung der Carbonsäuren im allgemeinen Alkalicarbonate wie beispielsweise Natrium- oder Kaliumcarbonat, oder organische Basen wie Trialkylamine, z.B. Triethylamin, N-Ethylmorpholin, N-Methylpiperidin oder Diisopropylethylamin, oder Dimethylaminopyridin, 1,8-Diazabicyclo[5.4.0]-undec-7-en (DBU) oder 1,5-Diazabicyclo[4.3.0]non-5-en (DBN). Bevorzugt ist Dimethylaminopyridin.

Die Base wird im allgemeinen in einer Menge von 0,01 mol bis 1 mol, bevorzugt von 0,05 mol bis 0,1 mol jeweils bezogen auf 1 mol der Verbindungen der allgemeinen Formeln (II) und (IX) eingesetzt.

Die Hilfsstoffe werden im allgemeinen in einer Menge von 1 mol bis 3 mol, bevorzugt von 1 mol bis 1,5 mol, jeweils bezogen auf 1 mol der Verbindungen der allgemeinen Formeln (II) und (IX) eingesetzt.

Die Reaktionstemperaturen für die Verfahren [A] und [B] können in einem größeren Bereich variiert werden. Im allgemeinen arbeitet man in einem Bereich von -20°C bis 200°C, bevorzugt von 0°C bis 110°C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B. außerdem, indem man Diastereomerengemische der Verbindungen der allgemeinen Formel (I), in welcher R¹ oder R³ für einen enantiomerenreinen chiralen Alkoholrest stehen, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen.

Die Veresterung der enantiomerenreinen Dihydropyridine erfolgt bevorzugt in Ethern wie Diethylether oder Tetrahydrofuran, in Dimethylformamid, Methylenchlorid, Chloroform, Acetonitril oder Toluol.

Die Aldehyde der allgemeinen Formel (II) sind an sich bekannt oder können nach üblichen Methoden hergestellt werden.

Ebenso sind die Acetessigester der allgemeinen Formeln (III) und (V) und die Enaminoderivate der allgemeinen Formeln (VI) und (VIII) bekannt.

Die reaktiven Säurederivate der allgemeinen Formel (IX) sind teilweise bekannt oder neu und können dann nach üblichen Methoden hergestellt werden.

Die Verbindungen der allgemeinen Formel (X) sind bekannt.

Die Verbindungen der allgemeinen Formeln (IV) und (VII) sind größenteils bekannt oder können nach üblichen Methoden hergestellt werden.

Die vorstehenden Herstellungsverfahren sind lediglich zur Verdeutlichung angegeben. Die Herstellung der Verbindungen der allgemeinen Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum.

Die erfindungsgemäßen Verbindungen sind Calciumkanalliganden mit Selektivität für L-Typ-Calciumkanäle des zentralen Nervensystems. Diese Selektivität läßt sich z.B. durch Vergleich der Bindungsaffinitäten zu DHP-Bindungstellen an Rattenhirn und Rattenherz zeigen.

Die Verbindungen beeinflussen positiv Lern- und Gedächtnisleistungen, wie ihre leistungsverbessernde Wirkung in typischen Lem- und Gedächtnismodellen wie Wasser-Labyrinth, Morris-Labyrinth, Passives Vermeiden oder Reminiszenztests in automatisierten Skinner-Boxen demonstriert.Sie besitzen ein antidepressives Potential, wie ihre Wirksamkeit im Rattenschwimmtest nach Porsolt belegt.

### Calcium-Flux

Zur Bestimmung des Calcium-Flux wird eine Suspension von kultivierten PC 12 Zellen verwendet. Die Zellen werden zusammen mit dem zu untersuchenden Wirkstoff bei 37°C in ein übliches Kulturmedium inkubiert. Zum Depolarisieren der Zellen wird ein Aktivierungsmedium mit einer hohen Kalium-Konzentration, das gleichzeitig radioaktives Calcium (⁴⁵Ca²⁺) enthält, zugegeben. Nach einem bestimmten Zeitintervall wird ein auf 0°C gekühltes Medium zugegeben um den Einstrom von radioaktivem Calcium in den Zellen zu stoppen. Anschließend wird die Radioaktivität in den geernteten und getrockneten Zellen bestimmt. Zur Festlegung der 0 %-Grenze des Hemmwertes wird Dimethylsulfoxid (DMSO) eingesetzt und der 100 % Hemmwert wird mit 10⁻⁶ mol/l Pimozid festgelegt.

### Bindungsassays:

Die Bindungsaffinitäten zu PN 200-110-Bindungsstellen im Rattenhirnen bzw. Rattenherzen wurden nach Rampe D.R., Rutledge A., Janis R.A., Triggle D.J.: Can. Journ. Physiol. Pharmacol. 65, (1987) 1452 bestimmt.

### Wasserlabyrinth:

Alte Wistar-Ratten (24 Monate) werden an die Startposition in einem mit kaltem (14-15°) Wasser gefüllten, durch senkrechte Barrieren unterteilten Plastiktank (120x50x40 cm) gesetzt. Um zu einer Leiter zu gelangen, die den Tieren das Entkommen aus dem Wasser ermöglicht, müssen sie um diese Barrieren herumschwimmen. Die Zeit, die zum Auffinden des Ausstiegs benötigt wird und die Zahl der Fehler auf dem Weg dorthin werden aufgezeichet. Dabei wird ein Fehler definiert als Schwimmen in eine Sackgasse oder Schwimmen über die Grenzlinie imaginärer Quadrate, in die der Tank unterteilt ist, in die Richtung fort vom Ausstieg.
Die Ratten bleiben bis zum Finden des Ausgangs, längstens aber 300 sec. im Labyrinth. Dann werden sie aufgenommen, getrocknet und unter einem Rotlicht gewärmt. Danach kehren sie in ihre Heimatkäfige zurück.
In einem typischen Experiment werden durch einen Vortest zwei äquivalente Tiergruppen (Placebo, Testsubstanz je n = 15) ermittelt. Anschließend durchlaufen die Tiere 6 Testsitzungen, zwei je Tag. Testsubstanzen bzw. Placebo werden 30 min. vor den Versuchen per os verabreicht. Maß für die lern- und gedächtnisverbessernde Wirkung der Testsubstanzen im Vergleich zu Placebo sind Verkürzung der Zeit bis zum Erreichen des Ausstiegs, Verringerung der Zahl der Fehler und Vergrößerung der Zahl der Tiere,die den Ausstieg überhaupt finden.

### Rattenschwimmtest nach Porsolt

Während eines Vortests werden junge Ratten in einen Glaszylinder (40 cm hoch, 20 cm Durchmesser) gesetzt, der 17 cm hoch mit Wasser von 25°C gefüllt ist. Nach 20 min im Wasser werden die Tiere herausgenommen und unter einer Lampe 30 min gewärmt. In diesem Vortest versuchen alle Ratten, aus dem Zylinder zu entkommen, bis sie nach etwa 15 min immobil verharren ("behavioral despair", Aufgabeverhalten). 24 h später beginnt die Testsitzung in der die Ratten wie am Vortag in den Glaszylinder gesetzt werden, jedoch diesmal nur 5 min. Die Zeitspannen, die die Ratten während dieser 5 min immobil verharren, werden aufgezeichnet. Dabei wird eine Ratte als immobil angesehen, die aufrecht im Wasser treibend nur noch minimale Bewegungen ausführt, um den Kopf über Wasser zu halten. Placebo bzw. Testsubstanzen (0,25, 0,5, 1, 5, 10 mg/kg; n = 6 je Gruppe) werden dreimal per os verabreicht: 23, 5 und 1 h vor der Testsitzung (1, 19, 23 h nach dem Vortest). Die antidepressive Wirkung der Testsubstanzen zeigt sich in der Reduktion der Immobilitätsdauer im Vergleich zu den Placebowerten.

Aufgrund ihrer pharmakologischen Eigenschaften können die erfindungsgemäßen Verbindungen für die Herstellung von Arzneimitteln zur Behandlung von zentral degenerativen Erkrankungen eingesetzt werden, wie sie z.B. auftreten bei Demenzen (Multiinfarktdemenz, MID, primär degenerativer Demenz PDD, prä- und seniler Alzheimerscher Krankheit, HIV-Demenz und andere Demenzformen), Parkinsonscher Krankheit oder amyotrophischer Lateralsklerose.

Weiterhin eignen sich die Wirkstoffe zur Behandlung von Hirnleistungsstörungen im Alter, des hirnorganischen Psychosyndroms (HOPS, Organic brain syndrom, OBS) und von altersbedingten Gedächtnisstörungen (age associated memory impairment, AAMI).

Die erfindungsgemäßen Verbindungen sind Ca²⁺-Antagonisten mit Selektivität für L-Typ-Ca²⁺-Kanäle des zentralen Nervensystems.

Diese Selektivität übertrifft die der bekannten cerebral wirksamen Ca²⁺-antagonistischen Dihydropyridine Nimodipin und Nicardipin. Dies zeigt sich z.B. beim Vergleich der Bindungsaffinitäten zu DHP-(PN-200 110)-Bindungsstellen in Rattenhirn und Rattenherz [vgl. Rampe, D.R., Rutledge, A., Janis, R.A., Triggle, D.J., Can. Journ. Physiol. Pharmacol. 65 (1987), 1452].

| Bsp.-Nr. | Kᵢ (Hirn) [nM] | Kᵢ (Herz) [nM] | Selektivität |
|---|---|---|---|
| Nimodipin | 2,4 | 4,6 | 1,9 |
| Nicardipin | 32 | 14 | 0,44 |
| 12 | 7,2 | 72 | 10 |
| 15 | 2,0 | 11,3 | 5,7 |
| 18 | 8,2 | 28 | 3,4 |
| 30 | 1,7 | 8,0 | 4,7 |
| 33 | 3,6 | 7,9 | 2,2 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 0,1 mg/kg bis 20 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung oder mit Molybdatophosphorsäurelösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in einem steigenden Ausmaß die polare Laufmittelkomponente zugemischt, bis das gewünschte Produkt eluiert wird (DC-Kontrolle).

Bei allen Produkten wurde bei zuletzt ca. 0,1 Torr das Lösemittel abdestilliert.

### Ausgangsverbindungen

### Beispiel I

### 2-Acetyl-3-(2-fluorphenyl)-2-propensäure-2-methoxyethylester

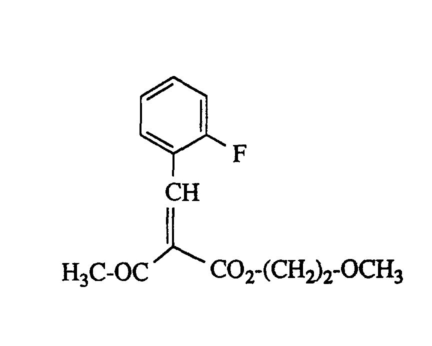

10 g (80 mmol) 2-Fluorbenzaldehyd werden mit 13 g (80 mmol) Acetessigsäure-2-methoxyethylester in 200 ml Isopropanol gelöst. Dazu gibt man eine frisch bereitete Lösung von 1,0 ml Piperidin und 0,5 ml Eisessig in 10 ml Isopropanol und rührt über Nacht bei 40°C. Man engt das Gemisch ein, nimmt in Toluol auf, engt erneut ein und reinigt durch Filtration über 300 ml Kieselgel (Laufmittel: Toluol / Essigester 100:1 - 10:1) zu 15 g der Zielverbindung als gelbes Öl, das unmittelbar weiter umgesetzt wird.

### Beispiel II

### 2-Acetyl-3-(2,4-difluorphenyl)-2-pmpensäure-2-methoxyethylester

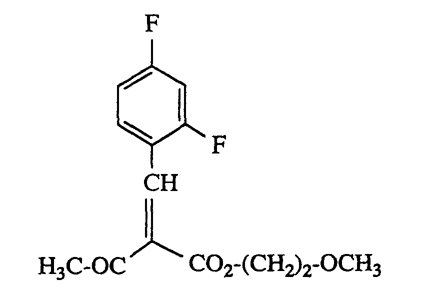

5,0 g (35 mmol) 2,4-Difluorbenzaldehyd werden mit 5,7 g (35 mmol) Acetessigsäure-2-methoxyethylester in 100 ml Isopropanol gelöst. Dazu gibt man eine frisch bereitete Lösung von 1,0 ml Piperidin und 0,5 ml Eisessig in 5 ml Isopropanol und rührt über Nacht bei 40°C. Man engt das Gemisch ein, nimmt in Toluol auf, engt erneut ein und reinigt durch Filtration über 100 ml Kieselgel (Laufmittel: Toluol / Essigester 100:1) zu 5 g der Zielverbindung als gelbes Öl, das unmittelbar weiter umgesetzt wird.

### Beispiel III

### 4-(2-Chlor-6-fluorphenyl)-1,4-dihydro-2,6-dimethylpyridin-3,5-dicarbonsäuremonocyclopentylester

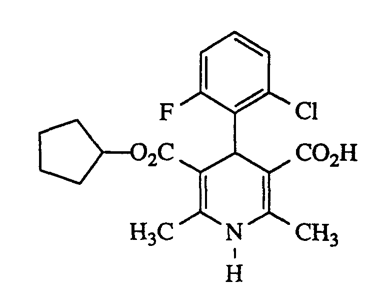

98 g (0,22 mol) 4-(2-Chlor-6-fluorphenyl)-1,4-dihydro-2,6-dimethylpyridin-3,5-dicarbonsäure-(2-cyanoethyl)-cyclopentylester werden in 400 ml 1,2-Dimethoxyethan gelöst und mit 400 ml 1 N Natronlauge über Nacht bei Zimmertemperatur gerührt. Man reduziert das Lösemittelvolumen auf etwa die Hälfte, wäscht mit Dichlormethan und säuert die wäßrige Phase mit 2 N Salzsäure an (pH = 2). Zweifache Extraktion mit Dichlormethan, Waschen der organischen Phasen mit Wasser, Trocknen über Natriumsulfat, Einengen und Kristallisation aus Ether ergibt 42 g der Zielverbindung als Feststoff vom Schmelzpunkt ca. 120°C (Zers.).

### Beispiel IV

### 4-(2-Chlor-6-fluorphenyl)-1,4-dihydro-5-(1-imidazolylcarbonyl)-2,6-dimethylpyridin-3-carbonsäurecyclopentylester

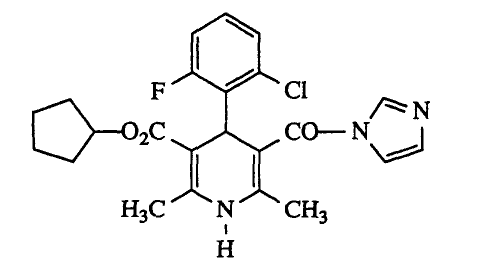

Zu 33,0 g (83 mmol) 4-(2-Chlor-6-fluorphenyl)-1,4-dihydro-2,6-dimethylpyridin-3,5-dicarbonsäuremonocyclopentylester in 350 ml Tetrahydrofuran gibt man 13,6 g (83 mmol) Carbonyldiimidazol und erhitzt 3 h zu Rückfluß. Dünnschichtchromatographische Kontrolle (Kieselgel, Toluol / Essigester 1:1) zeigt vollständigen Umsatz, woraufhin das Reaktionsgemisch eingeengt, in Essigester aufgenommen, zweimal mit Wasser gewaschen, über Natriumsulfat getrocknet und erneut eingeengt wird. Aus Ether fällt die Zielverbindung in Form weißer Kristalle vom Schmp. 150°C.
Ausbeute: 29,7 g

### Herstellungsbeispiele

### Beispiel 1

### Isopropyl-(2-methoxyethyl)-4-(2-fluorphenyl)-1,4-dihydro-2,6-dimethylpyridin-3,5-dicarboxylat

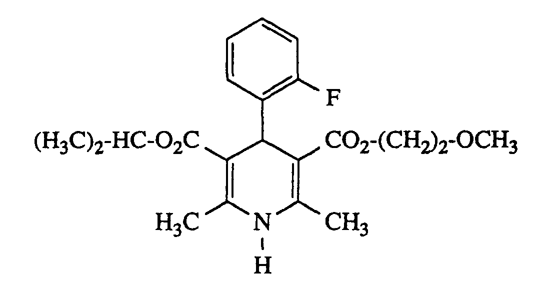

4,0 g (ca 15 mmol) der Verbindung aus Beispiel I werden mit 2,2 g (15 mmol) 3-Amino-2-butensäure-isopropylester in 100 ml Isopropanol über Nacht zum Rückfluß erhitzt. Nachdem dünnschichtchromatographische Kontrolle (SiO₂, Toluol / Essigester 5:1) vollständigen Umsatz zeigt, wird das Reaktionsgemisch eingeengt, mit Toluol aufgenommen, erneut eingeengt und sodann durch Filtration über Kieselgel (Laufinittel: Toluol / Essigester 100:1 - 5:1) gereinigt. Man erhält ein kristallisierendes gelbes Öl, das bei ca. -15°C aus Methanol unkristallisiert wird. Man erhält 2,8 g (48%) der Zielverbindung.
Smp.: 99-100°C

### Beispiel 2

### (-)-Cycloheptyl-(2-methoxyethyl)-4-(3-cyanophenyl)-1,4-dihydro-2,6-dimethyl-pyridin-3,5-dicarboxylat

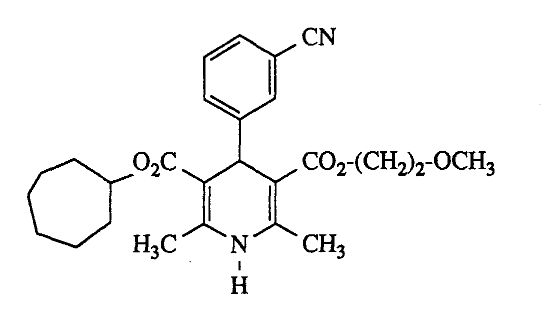

5,0 g (14 mmol) (-)-4-(3-Cyanophenyl)-1,4-dihydro-2,6-dimethylpyridin-3,5-dicarbonsäure-mono-(2-methoxyethyl)-ester [erhältlich durch chromatographische Trennung der racemischen Monocarbonsäure an chiralen stationären Phasen] werden in 50 ml Tetrahydrofuran 30 min über Molekularsieb (4 A) gerührt. Dann gibt man 2,3 g (14 mmol) Carbonyldiimidazol zu und erhitzt 1 h zum Rückfluß, filtriert vom Molekularsieb ab und engt ein. Man nimmt den Rückstand in 30 ml Cycloheptanol auf und erhitzt nach Zugabe einer Spatelspitze N,N-Dimethyl-4-aminopyridin für 6 h auf 100°C. Wiederholte Chromatographie an Kieselgel in Toluol / Essigester, Dichlormethan / Isopropanol und Cyclohexan / Essigester-Mischungen und Kristallisation aus Diisopropylether / Cyclohexan ergibt 1,0 g (16%) der Zielverbindung als weiße Kristalle.
Schmp.: 98-99°C
α²⁰_{D} = -24,2 (c = 0,9, CHCl₃)

Die in der Tabelle 1 aufgeführten Verbindungen werden in Analogie zu den Beispielen 1 und 2 erhalten, oder indem man die racemischen Produkte einer chromatographischen Trennung an chiralen stationären Phasen (Chiralcel und Chiralpak, Daicel) in die enantiomerenreinen Zielverbindungen unterzieht:

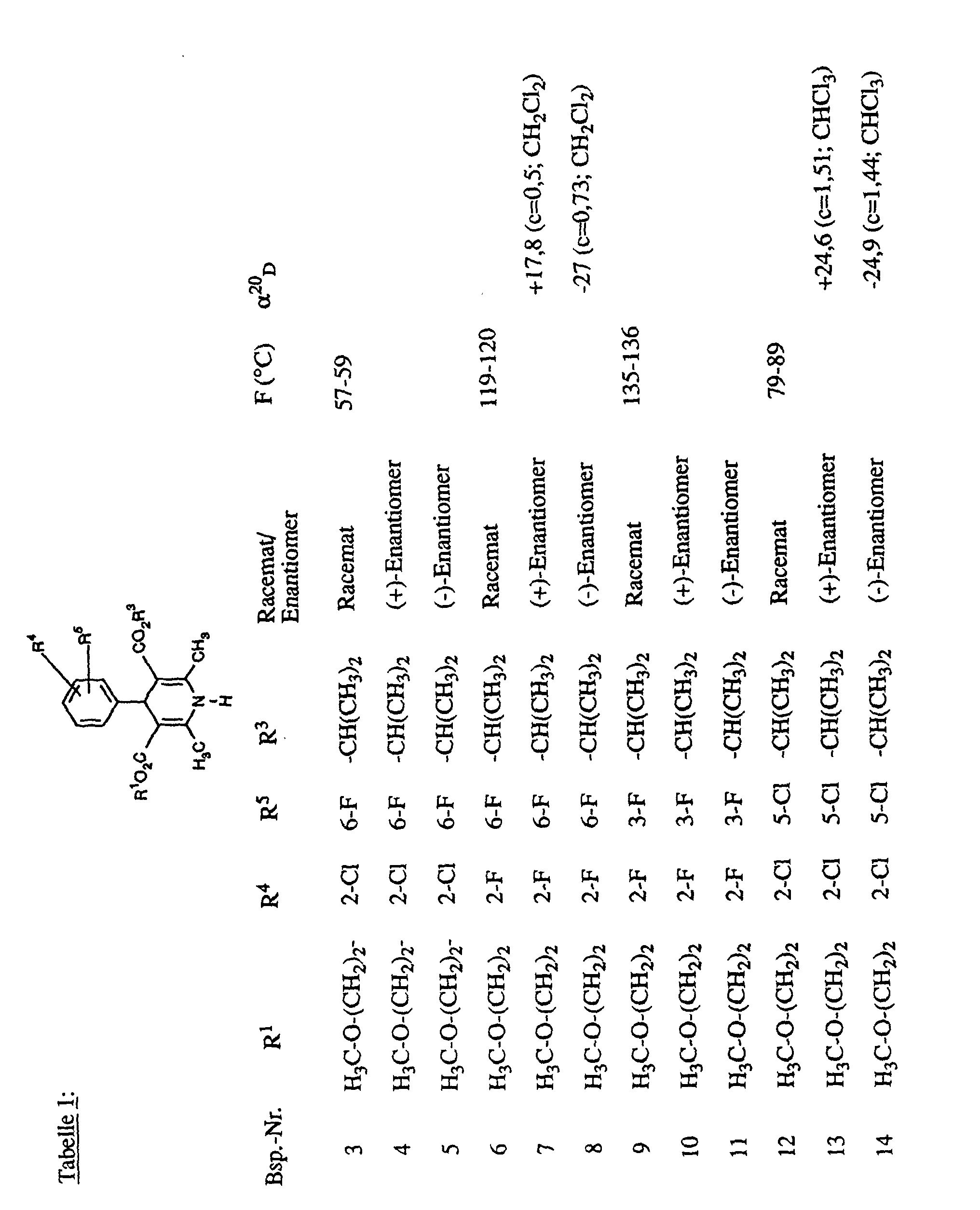

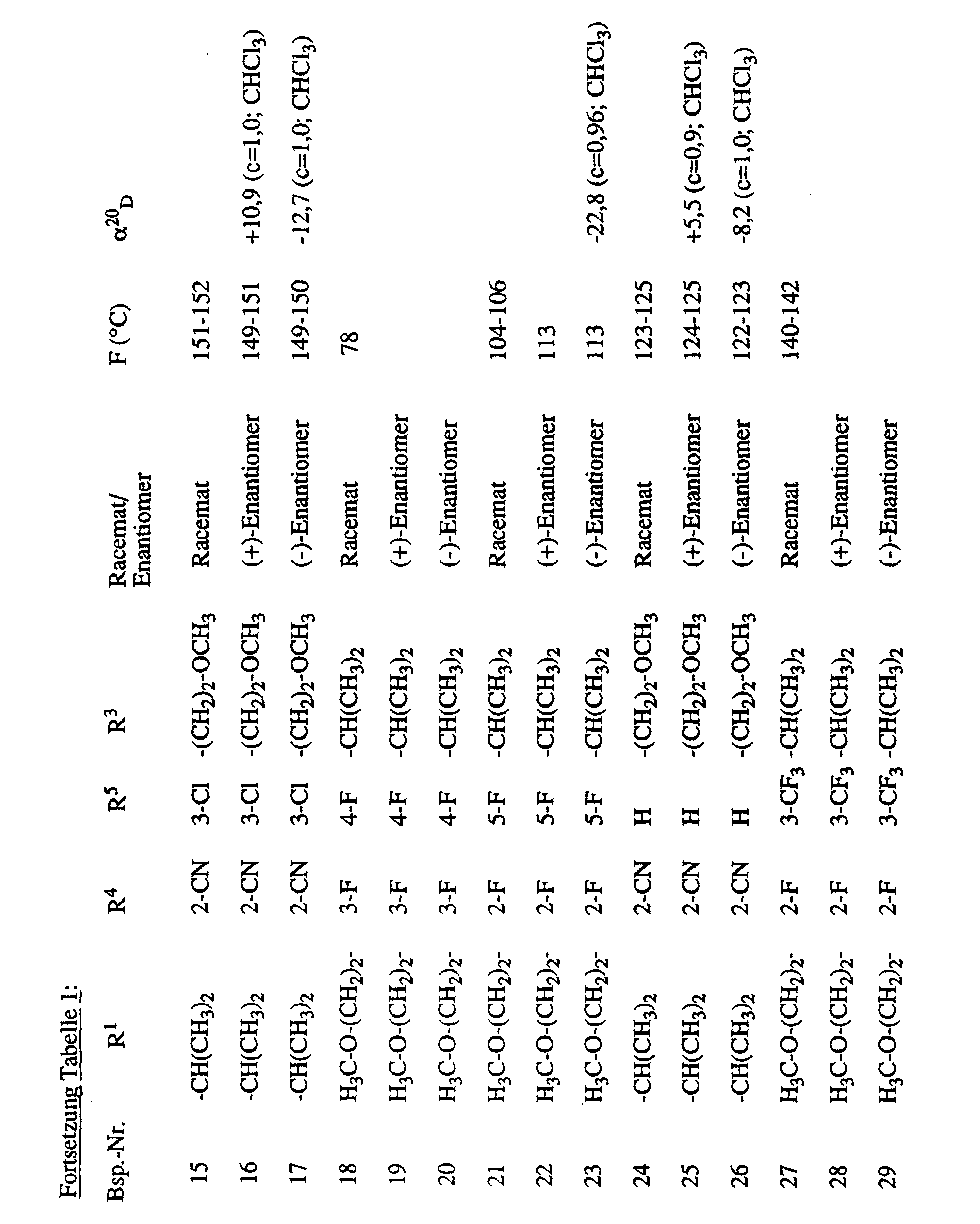

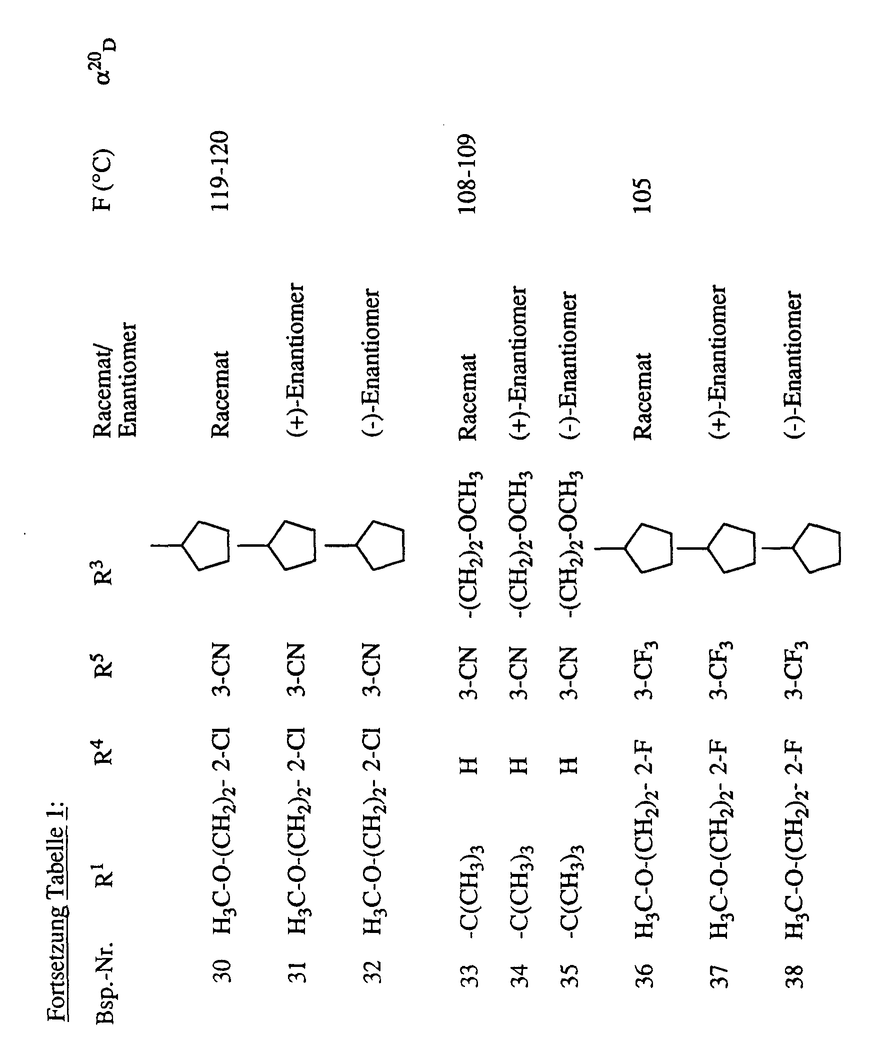

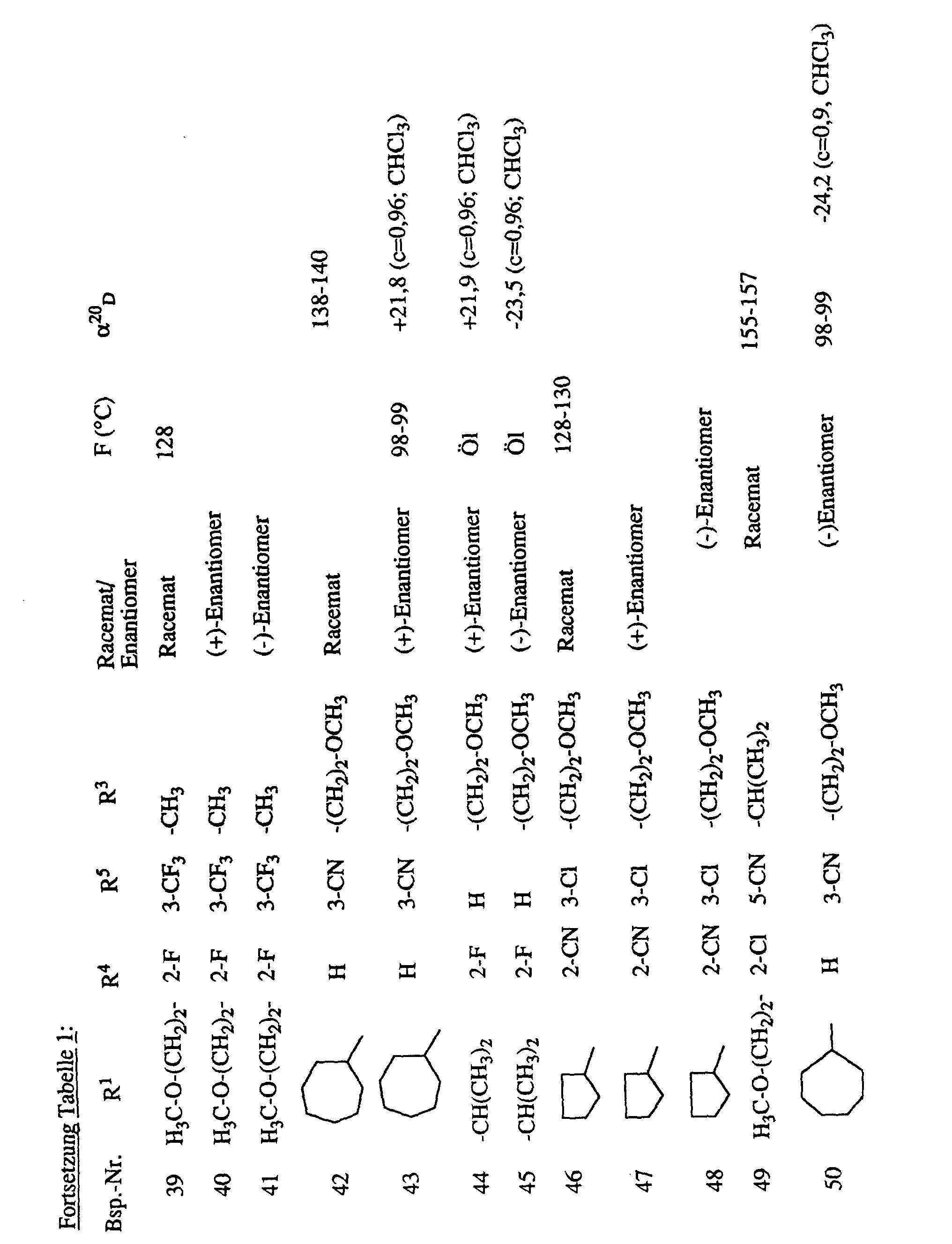

### Beispiel 51

### 5-(2,4-Difluorphenyl)-1,4-dihydro-2,6-dimethylpyridin-3,5-dicarbonsäure-(2-methoxyethyl)-isopropylester

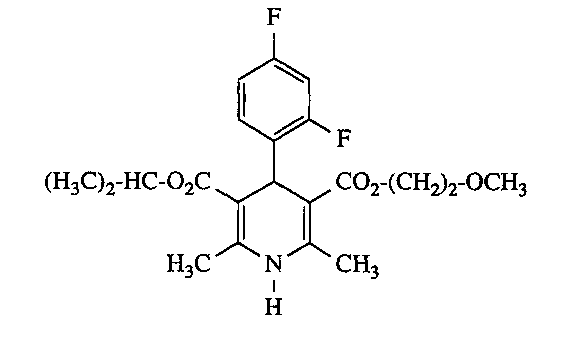

5,0 g (ca. 18 mmol) der Verbindung aus Beispiel IV werden mit 2,5 g (18 mmol) 3-Amino-2-butensäure-isopropylester in 50 ml Isopropanol über Nacht zum Rückfluß erhitzt. Nachdem dünnschichtchromatographische Kontrolle (SiO₂, Toluol / Essigester 5:1) vollständigen Umsatz zeigt, wird das Reaktionsgemisch eingeengt, mit Toluol aufgenommen, erneut eingeengt und sodann durch Kristallisation aus wenig Methanol gereinigt. Man erhält 2,8 g (39%) der Zielverbindung.
Smp.: 123-126°C

In Analogie zur Vorschrift der Beispiele 1, 2 und 51 werden die in den Tabellen 2, 3, 4, 5, 6 und 7 aufgeführten Verbindungen hergestellt:

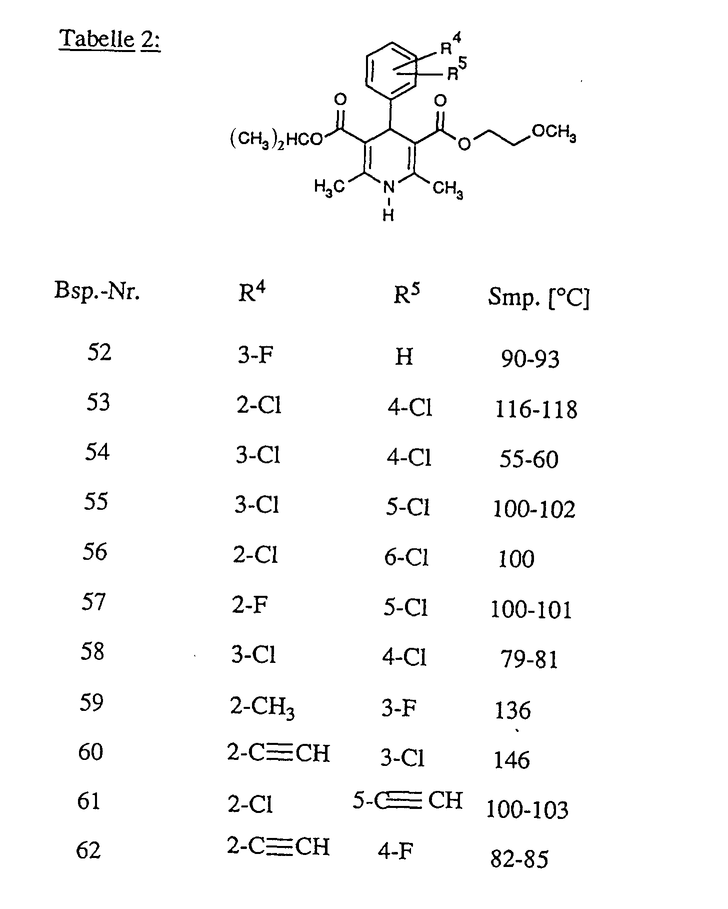

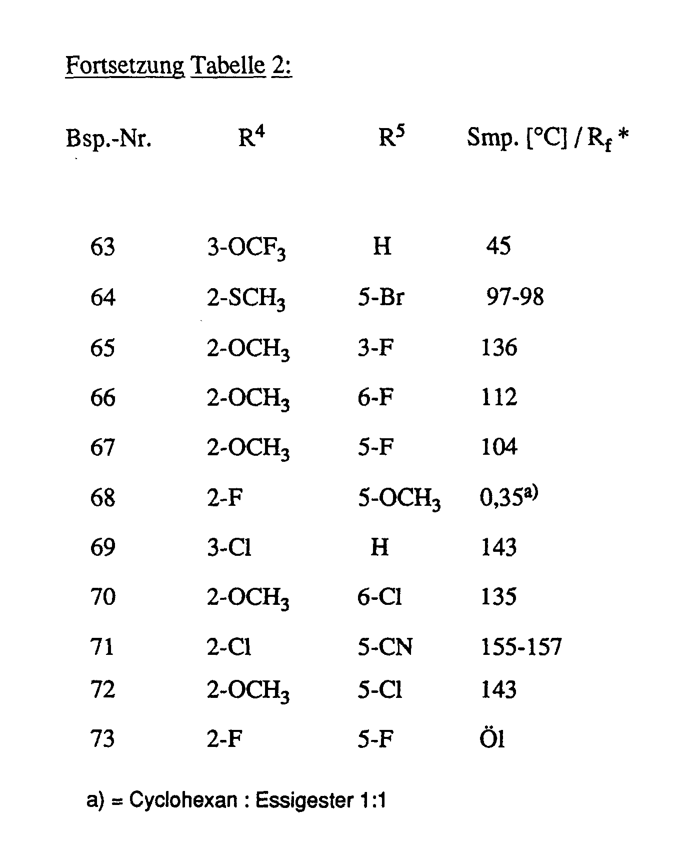

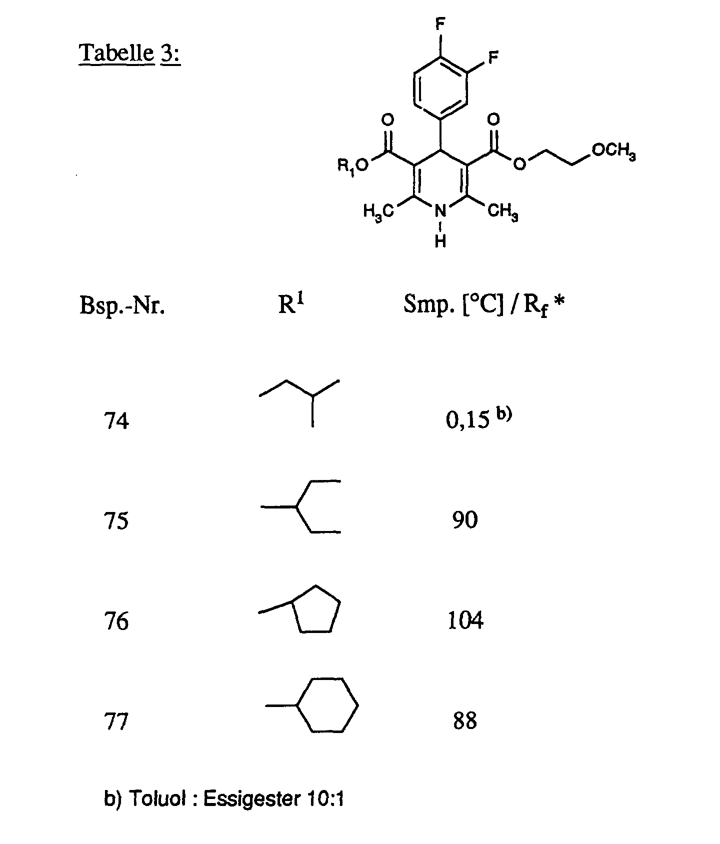

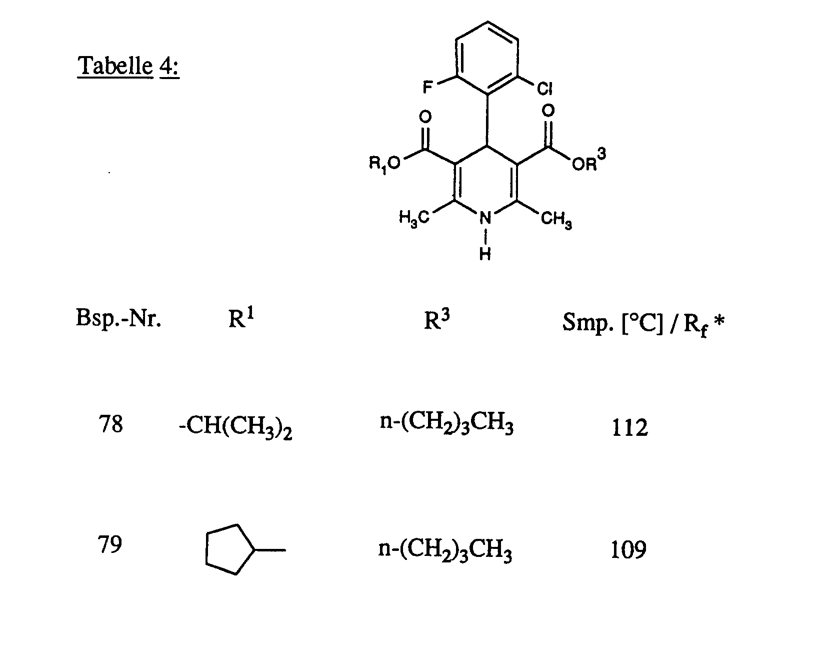

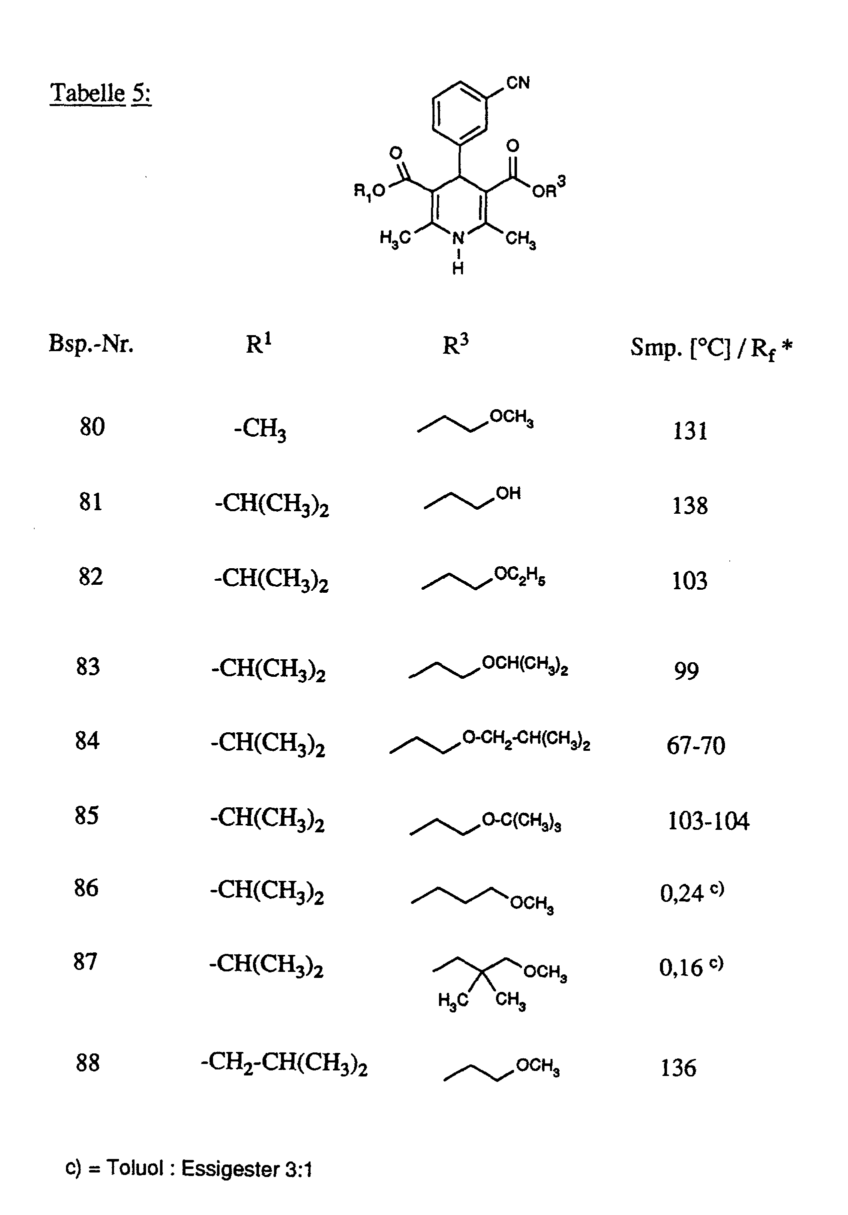

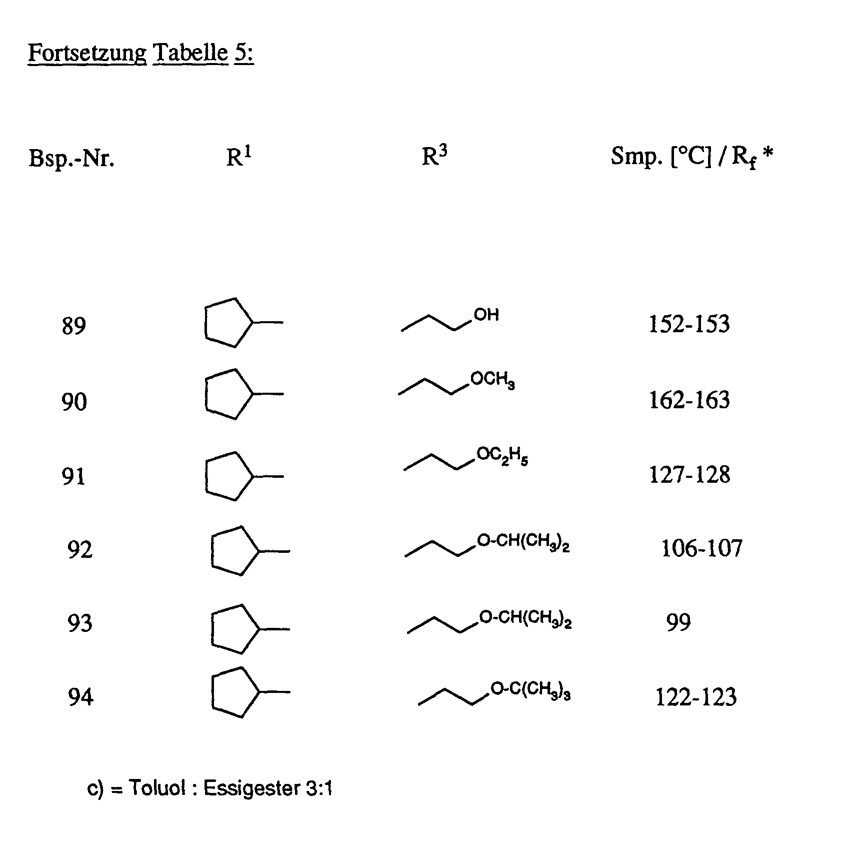

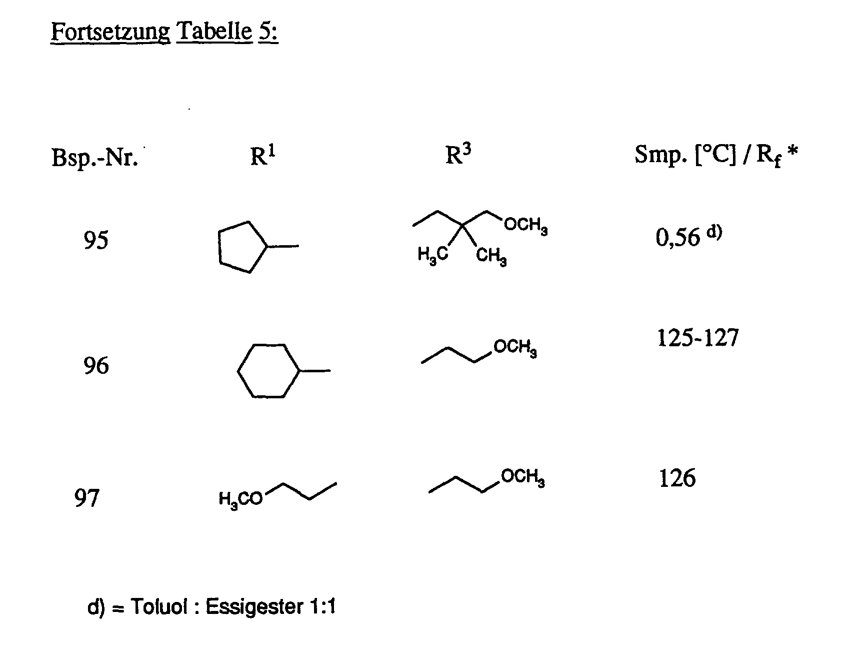

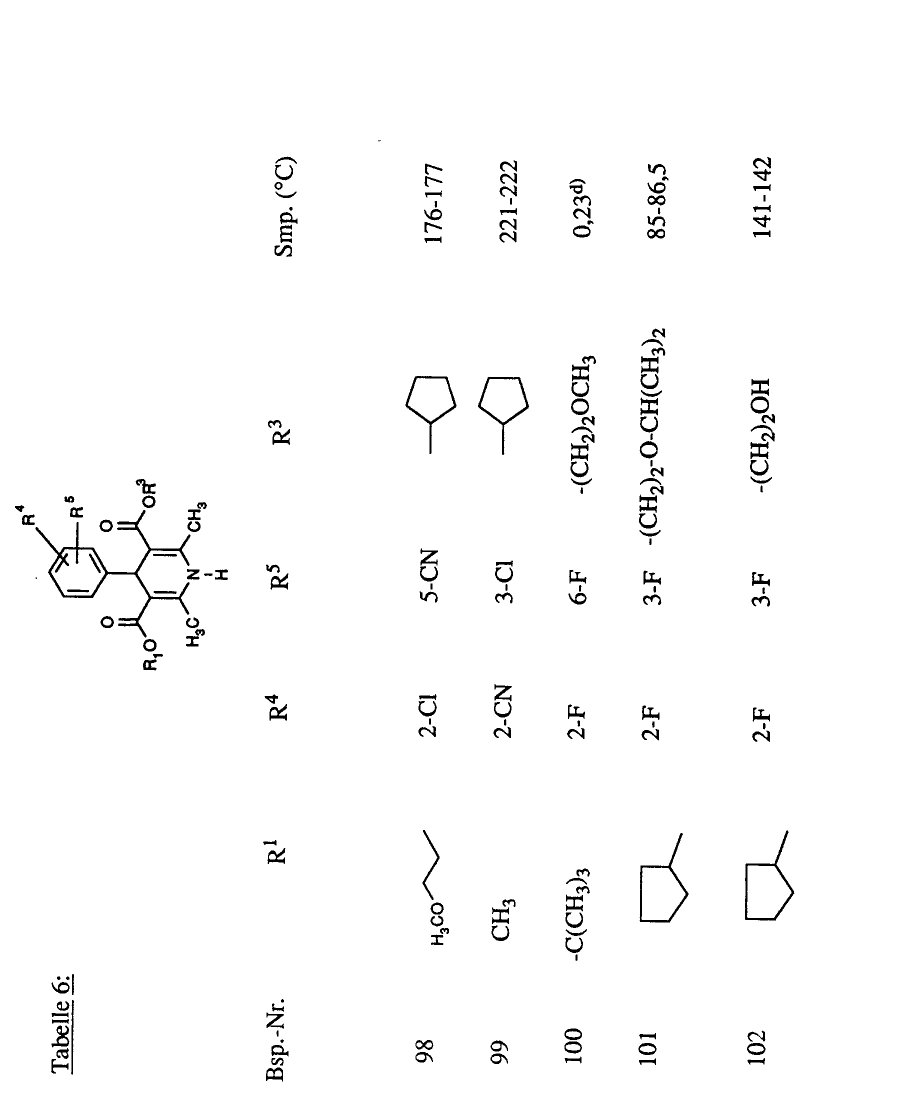

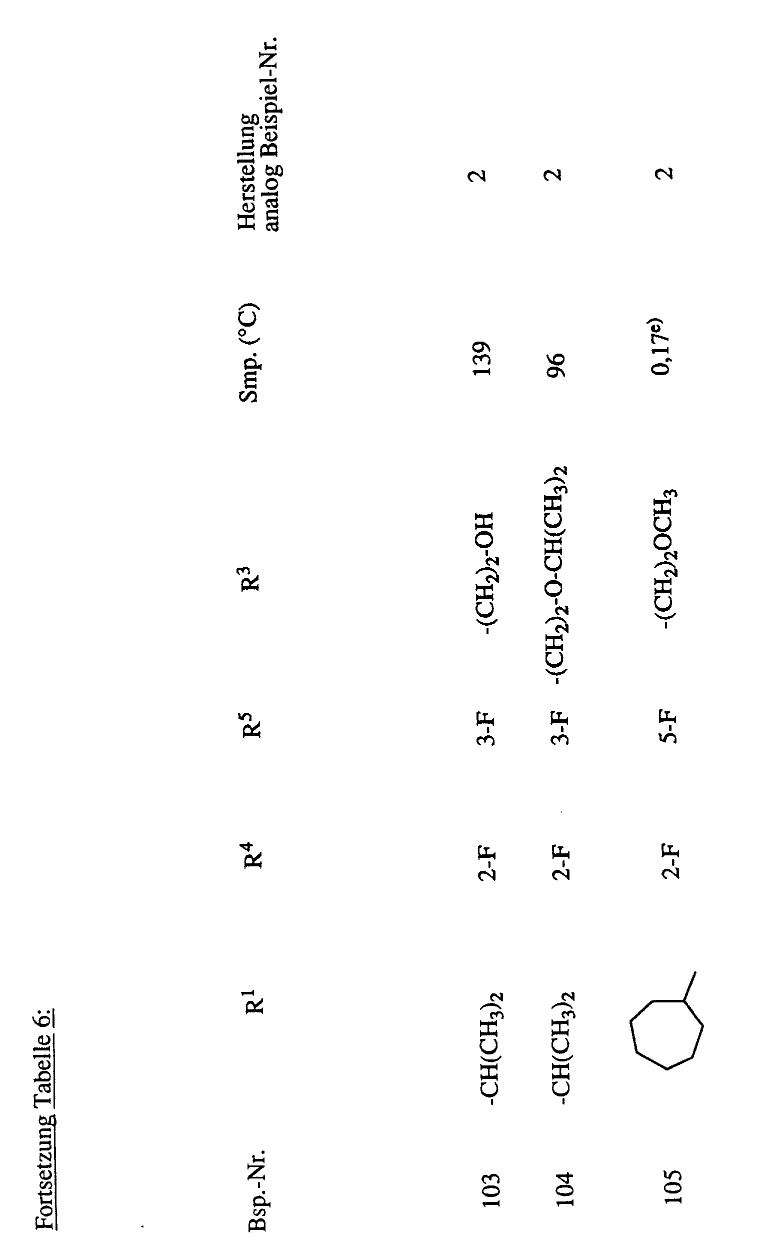

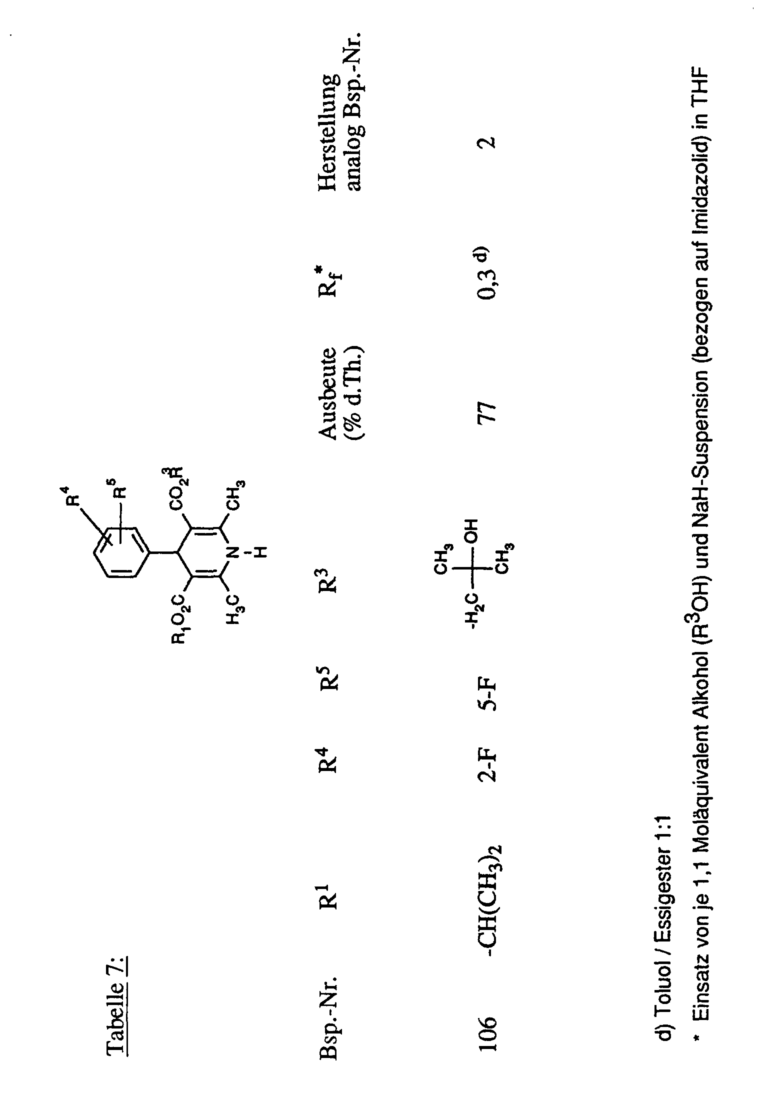

### Beispiel 107

### (-)-4-(3-Cyanophenyl)-1,4-dihydro-2,6-dimethylpyridin-3,5-dicarbonsäuremethyl-(2-methoxyethyl)-ester

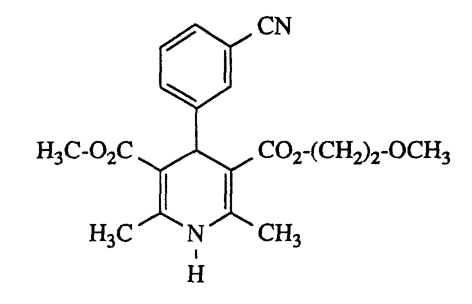

3,6 g (10 mmol) (-)-4-(3-Cyanophenyl)-1,4-dihydro-2,6-dimethylpyridin-3,5-dicarbonsäure-(2-methoxyethyl)-ester werden in 40 ml Tetrahydrofuran 30 min über Molekularsieb (4 A) gerührt. Dann gibt man 1,6 g (10 mmol) Carbonyldiimidazol zu und erhitzt 0,5 h auf 60°C, filtriert vom Molekularsieb ab und engt ein. Man nimmt den Rückstand in 40 ml Methanol auf und erhitzt für 6 h auf 80°C. Wiederholte Chromatographie an Kieselgel mit Toluol / Essigester-Mischungen (1:0 bis 3:1) ergibt 1,4 g ölige Substanz, die durch Verreiben mit Diethylether zur Kristallisation gebracht werden. 1,1 g (30%) farblose Kristalle vom Schmp. 103-104°C werden so erhalten.
α²⁰_{D} = -7,0 (c = 1,2; CHCl₃)

Die in der Tabelle 8 aufgeführten Verbindungen werden erhalten in Analogie zu Beispiel 107 oder indem man die racemischen Produkte einer chromatographischen Trennung an chiralen stationären Phasen (Chiralcel und Chiralpak, Daicel) in die enantiomerenreinen Zielverbindungen unterzieht (in Folgenden A*):

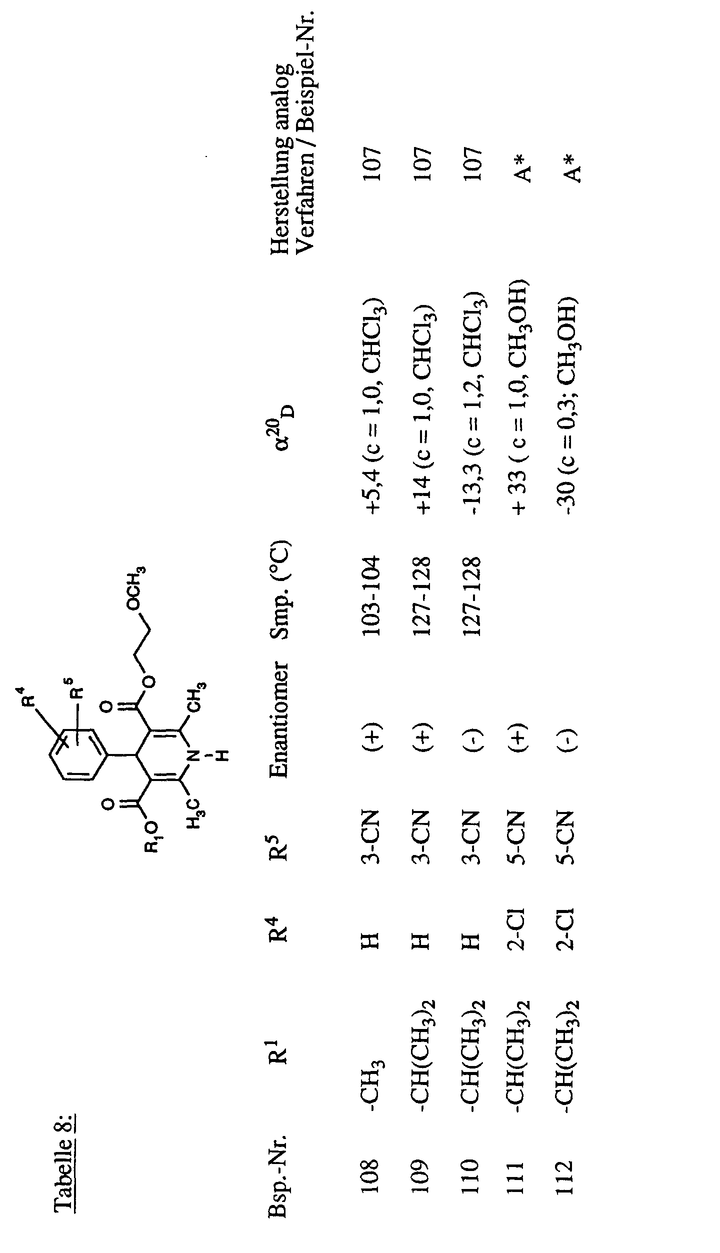

### Beispiel 113

### Bis-(2-methoxyethyl)-4-(2-fluor-3-trifluormethylphenyl)-1,4-dihydro-2,6-dimethylpyridin-3,5-dicarboxylat

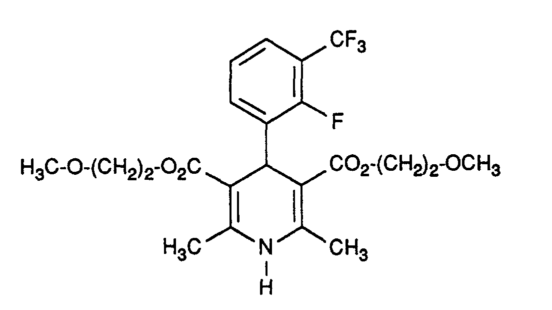

Zu einer Lösung von 3,0 g (15,6 mmol) 2-Fluor-3-trifluormethylbenzaldehyd in 50 ml Dioxan gibt man 5 ml einer 25%igen Ammoniaklösung und 5,0 g (31,2 mmol) Acetessigsäure-2-methoxyethylester und erhitzt zum Rückfluß, bis DC-Kontrolle (Kieselgel, Toluol / Essigester 5:1) vollständigen Umsatz anzeigt. Man engt ein, nimmt zweimal in Toluol auf und engt erneut ein. Kristallisation aus Toluol ergibt 1,4 g (19%) der Zielverbindung vom Schmelzpunkt 148°C.

In Analogie zur Vorschrift des Beispiels 113 werden die in Tabelle 9 aufgeführten Beispiele hergestellt:

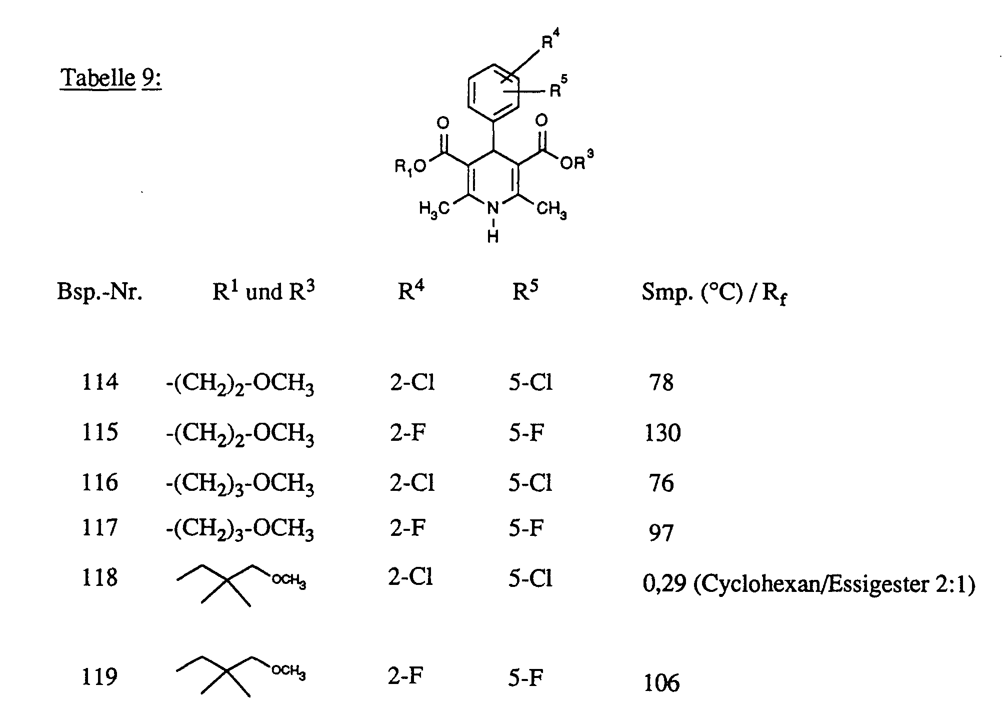

Die in Tabelle 10 aufgeführten Verbindungen werden in Analogie zur Vorschrift des Beispiels 107 aus dem entsprechenden Imidazolid hergestellt:

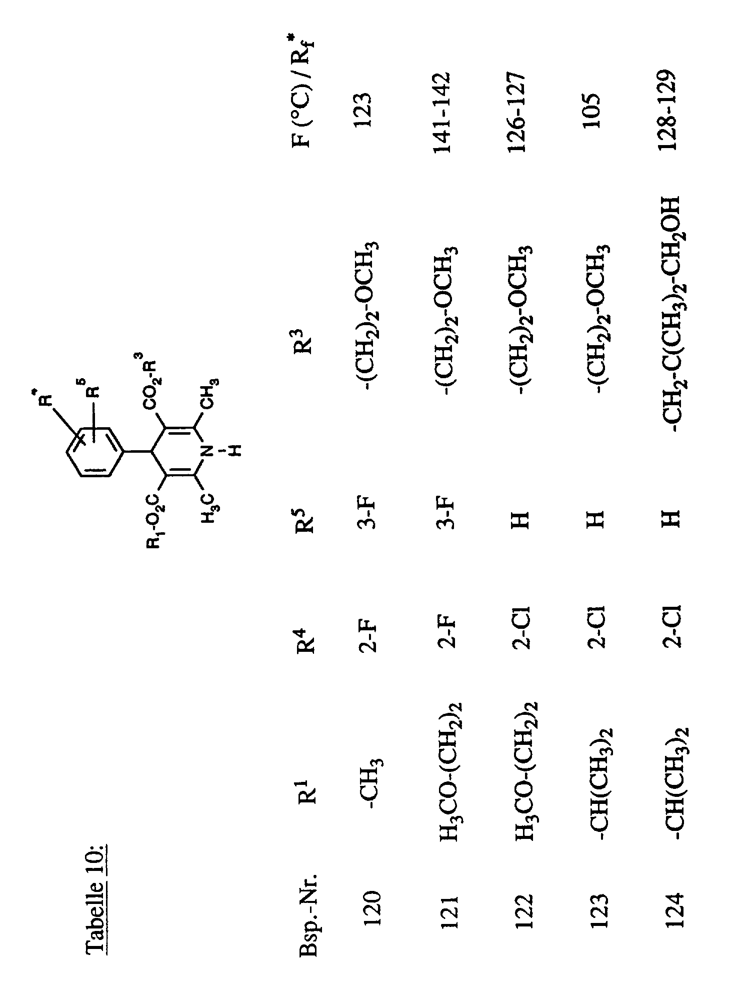

## Patentansprüche

1. Verwendung von Verbindungen der allgemeinen Formel 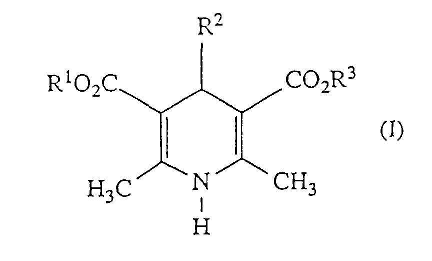 in welcher,
R¹ und R³ gleich oder verschieden sind und für geradkettiges oder verzweigtes Alkyl mit bis zu 8 Kohlenstoffatomen stehen, das gegebenenfalls durch geradkettiges oder verzweigtes Alkoxy mit bis zu 6 Kohlenstoffatomen oder Hydroxy substituiert ist, oder für Cycloalkyl mit 3 bis 7 Kohlenstoffatomen stehen,
und R² für den Rest 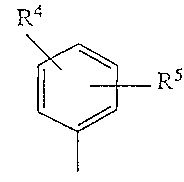 steht,
worin
R⁴ und R⁵ gleich oder verschieden sind und für Halogen, Cyano, Ethinyl, Trifluormethoxy, Methyl, Methylthio, Trifluormethyl oder für geradkettiges oder verzweigtes Alkoxy mit bis zu 4 Kohlenstoffatomen stehen,
oder
R⁴ oder R⁵ für Wasserstoff steht
zur Herstelllung eines Arzneimittels zur Behandlung von zentral degenerativen Erkrankungen oder Hirnleistungsstörungen.

2. Verwendung nach Anspruch 1, wobei
R¹ und R³ gleich oder verschieden sind und für geradkettiges oder verzweigtes Alkyl mit bis zu 8 Kohlenstoffatomen stehen, das gegebenenfalls durch geradkettiges oder verzweigtes Alkoxy mit bis zu 5 Kohlenstoffatomen oder Hydroxy substituiert ist, oder für Cyclopropyl, Cyclopentyl, Cyclohexyl oder Cycloheptyl stehen,
und R² für den Rest 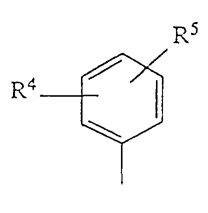 steht,
worin
R⁴ und R⁵ gleich oder verschieden sind und für Fluor, Chlor, Brom, Cyano, Ethinyl, Trifluormethoxy, Methyl, Methylthio, Trifluormethyl oder für geradkettiges oder verzweigtes Alkoxy mit bis zu 3 Kohlenstoffatomen stehen,
oder
R⁴ oder R⁵ für Wasserstoff steht.

3. Verwendung nach Anspruch 1, wobei
R¹ und R³ gleich oder verschieden sind und für geradkettiges oder verzweigtes Alkyl mit bis zu 8 Kohlenstoffatomen stehen, das gegebenenfalls durch Methoxy oder Hydroxy substituiert ist, oder für Cyclopentyl, Cyclopropyl, Cyclohexyl oder Cycloheptyl stehen,
und R² für den Rest 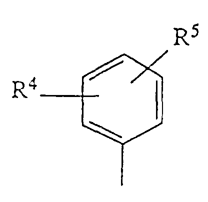 steht,
in welcher
R⁴ und R⁵ gleich oder verschieden sind und für Fluor, Chlor, Brom, Cyano, Ethinyl, Trifluormethoxy, Methyl, Methylthio, Trifluormethyl oder für geradkettiges oder verzweigtes Alkoxy mit bis zu 3 Kohlenstoffatomen stehen,
oder
R⁴ oder R⁵ für Wasserstoff steht.

4. Verwendung nach Anspruch 1, wobei
wobei
R³ für den Rest -(CH₂)ₙ-OR⁶ steht, worin n für eine Zahl von 2 bis 4 steht und R⁶ für Wasserstoff oder Alkyl mit 1 bis 4 C-Atomen steht und
R¹ mit R³ gleich oder verschieden ist und für Cyclopropyl, Cyclopentyl, Cyclohexyl oder Cycloheptyl steht oder für Alkyl mit bis zu 8 C-Atomen steht, welches gegebenenfalls durch Hydroxy oder Alkoxy mit 1 bis 4 C-Atomen substituiert ist.
und
R² die in Anspruch 3 angegebene Bedeutung hat.

5. Verwendung nach Anspruch 1, wobei
R¹ und R³ die in Anspruch 4 angegebene Bedeutung haben, und
R² für einen Phenylrest steht, der in 2- und 3-Position gleich oder verschieden durch Fluor, Chlor, Cyano oder CF₃ substituiert ist, wobei 2,3-Dichlorphenyl ausgeschlossen ist.

6. Verwendung nach Anspruch 4, in welcher
R³ für den Rest -CH₂-CH₂-OCH₃ steht, und R¹ und R² die in Anspruch 4 angegebene Bedeutung haben.

7. Verfahren zur Herstellung von Phenyl-substituierten 1,4-Dihydropyridinen nach Anspruch 8, **dadurch gekennzeichnet, dass** man
[A] Aldehyde der allgemeinen Formel (II)
R²-CHO (II)
in welcher
R² die in Anspruch 8 angegebene Bedeutung hat,
zunächst mit Acetessigestern der allgemeinen Formel (III)
H₃C-CO-CH₂-CO₂R¹ (III)
in welcher
R¹ die in Anspruch 8 angegebene Bedeutung hat,
gegebenenfalls unter Isolierung der entsprechenden Ylidenverbindungen der allgemeinen Formel (IV) 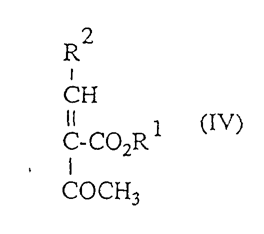 umsetzt und anschließend entweder mit Verbindungen der allgemeinen Formel (V)
CH₃-CO-CH₂-CO₂R³ (V)
in welcher
R³ die in Anspruch 8 angegebene Bedeutung hat,
in inerten Lösemitteln, in Anwesenheit von Ammoniak oder Ammoniumsalzen,
oder direkt mit Enaminoderivaten der allgemeinen Formel (VI) 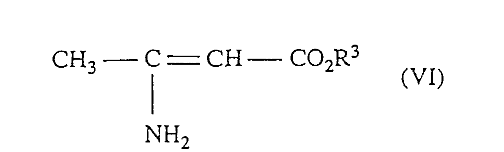 in welcher
R³ die oben angegebene Bedeutung hat,
umsetzt,
oder
[B] die Aldehyde der allgemeinen Formel (II) zunächst mit den Verbindungen der allgemeinen Formel (V), gegebenenfalls unter Isolierung der Ylidenverbindungen der allgemeinen Formel (VII) 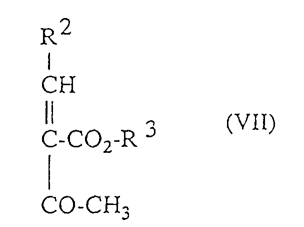 in welcher
R² und R³ die oben angegebene Bedeutung haben,
umsetzt und in einem nächsten Schritt mit den Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII) 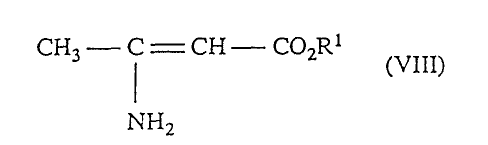 in welcher
R¹ die oben angegebene Bedeutung hat,
umsetzt,
oder
[C] Verbindungen der allgemeinen Formel (IX) 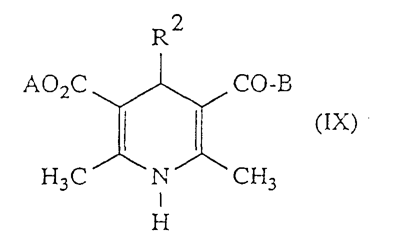
in welcher
R² die oben angegebene Bedeutung hat,
A die oben angegebene Bedeutung von R¹ oder R³ hat
und
B zusammen mit der -CO-Gruppe eine reaktives Carbonsäurederivat bildet,
in inerten Lösemitteln, in Anwesenheit einer Base,
mit Verbindungen der allgemeinen Formel (X)
R⁶-OH (X)
in welcher
R⁶ die oben angegebene Bedeutung von R¹ oder R³ hat,
umsetzt,
und im Fall der reinen Ester-Enantiomere, die enantiomerenreinen Carbonsäuren, gegebenenfalls zunächst über die Stufe eines reaktiven Säurederivates, mit den entsprechenden Alkoholen umsetzt.

8. Verbindungen der allgemeinen Formel (I) 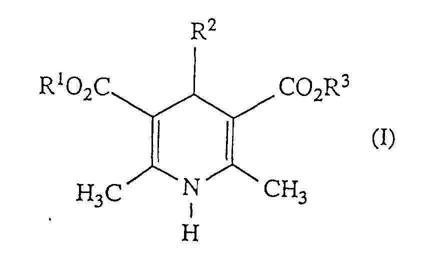 in welcher
R² für den Rest 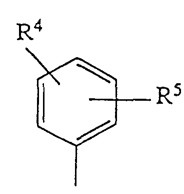 steht,
ausgewählt aus einer Gruppe von Verbindungen, in der die Substituenten R¹, R³, R⁴ und R⁵ die in der folgenden Tabelle aufgeführten Tabelle aufgeführten Bedeutungen haben:
| **R**^{**1**} | **R**^{**3**} | **R**^{**4**} | **R**^{**5**} | **Racemat, (+)-, (-)-Enantiomer** |
|---|---|---|---|---|
| isopropyl | CH₂CH₂OCH₃ | 2-F | H | Racemat ; |
| cycloheptyl | CH₂CH₂OCH₃ | 3-CN | H | (-) ; |
| isopropyl | CH₂CH₂OCH₃ | 2-Cl | 6-F | Racemat ; |
| isopropyl | CH₂CH₂OCH₃ | 2-Cl | 6-F | (+) ; |
| isopropyl | CH₂CH₂OCH₃ | 2-Cl | 6-F | (-) ; |
| isopropyl | CH₂CH₂OCH₃ | 2-F | 6-F | Racemat ; |
| isopropyl | CH₂CH₂OCH₃ | 2-F | 6-F | (+) ; |
| isopropyl | CH₂CH₂OCH₃ | 2-F | 6-F | (-) ; |
| isopropyl | CH₂CH₂OCH₃ | 2-F | 3-F | Racemat ; |
| isopropyl | CH₂CH₂OCH₃ | 2-F | 3-F | (+) ; |
| isopropyl | CH₂CH₂OCH₃ | 2-F | 3-F | (-) ; |
| isopropyl | CH₂CH₂OCH₃ | 2-Cl | 5-Cl | Racemat ; |
| isopropyl | CH₂CH₂OCH₃ | 2-Cl | 5-Cl | (+) ; |
| isopropyl | CH₂CH₂OCH₃ | 2-Cl | 5-Cl | (-) ; |
| isopropyl | CH₂CH₂OCH₃ | 2-CN | 3-Cl | Racemat ; |
| isopropyl | CH₂CH₂OCH₃ | 2-CN | 3-Cl | (+) ; |
| isopropyl | CH₂CH₂OCH₃ | 2-CN | 3-Cl | (-) ; |
| isopropyl | CH₂CH₂OCH₃ | 3-F | 4-F | Racemat ; |
| isopropyl | CH₂CH₂OCH₃ | 3-F | 4-F | (+) ; |
| isopropyl | CH₂CH₂OCH₃ | 3-F | 4-F | (-) ; |
| isopropyl | CH₂CH₂OCH₃ | 2-F | 5-F | Racemat ; |
| isopropyl | CH₂CH₂OCH₃ | 2-F | 5-F | (+) ; |
| isopropyl | CH₂CH₂OCH₃ | 2-F | 5-F | (-) ; |
| isopropyl | CH₂CH₂OCH₃ | 2-CN | H | Racemat ; |
| isopropyl | CH₂CH₂OCH₃ | 2-CN | H | (+) ; |
| isopropyl | CH₂CH₂OCH₃ | 2-CN | H | (-) ; |
| isopropyl | CH₂CH₂OCH₃ | 2-F | 3-CF₃ | Racemat ; |
| isopropyl | CH₂CH₂OCH₃ | 2-F | 3-CF₃ | (+) ; |
| isopropyl | CH₂CH₂OCH₃ | 2-F | 3-CF₃ | (-) ; |
| cyclopentyl | CH₂CH₂OCH₃ | 2-Cl | 3-CN | Racemat ; |
| cyclopentyl | CH₂CH₂OCH₃ | 2-Cl | 3-CN | (+) ; |
| cyclopentyl | CH₂CH₂OCH₃ | 2-Cl | 3-CN | (-) ; |
| t-butyl | CH₂CH₂OCH₃ | 3-CN | H | Racemat ; |
| t-butyl | CH₂CH₂OCH₃ | 3-CN | H | (+) ; |
| t-butyl | CH₂CH₂OCH₃ | 3-CN | H | (-) ; |
| cyclopentyl | CH₂CH₂OCH₃ | 2-F | 3-CF₃ | Racemat ; |
| cyclopentyl | CH₂CH₂OCH₃ | 2-F | 3-CF₃ | (+) ; |
| cyclopentyl | CH₂CH₂OCH₃ | 2-F | 3-CF₃ | (-) ; |
| methyl | CH₂CH₂OCH₃ | 2-F | 3-CF₃ | Racemat ; |
| Methyl | CH₂CH₂OCH₃ | 2-F | 3-CF₃ | (+) ; |
| methyl | CH₂CH₂OCH₃ | 2-F | 3-CF₃ | (-) ; |
| cycloheptyl | CH₂CH₂OCH₃ | 3-CN | H | Racemat ; |
| cycloheptyl | CH₂CH₂OCH₃ | 3-CN | H | (+) ; |
| isopropyl | CH₂CH₂OCH₃ | 2-F | H | (+) ; |
| isopropyl | CH₂CH₂OCH₃ | 2-F | H | (-) ; |
| cyclopentyl | CH₂CH₂OCH₃ | 2-CN | 3-Cl | Racemat ; |
| cyclopentyl | CH₂CH₂OCH₃ | 2-CN | 3-Cl | (+) ; |
| cyclopentyl | CH₂CH₂OCH₃ | 2-CN | 3-Cl | (-) ; |
| isopropyl | CH₂CH₂OCH₃ | 2-Cl | 5-CN | Racemat ; |
| cycloheptyl | CH₂CH₂OCH₃ | 3-CN | H | (-) ; |
| isopropyl | CH₂CH₂OCH₃ | 2-F | 4-F | Racemat ; |
| isopropyl | CH₂CH₂OCH₃ | 3-F | H | Racemat ; |
| isopropyl | CH₂CH₂OCH₃ | 2-Cl | 4-Cl | Racemat ; |
| isopropyl | CH₂CH₂OCH₃ | 3-Cl | 4-Cl | Racemat ; |
| isopropyl | CH₂CH₂OCH₃ | 3-Cl | 5-Cl | Racemat ; |
| isopropyl | CH₂CH₂OCH₃ | 2-Cl | 6-Cl | Racemat ; |
| isopropyl | CH₂CH₂OCH₃ | 2-F | 5-Cl | Racemat ; |
| isopropyl | CH₂CH₂OCH₃ | 3-Cl | 4-Cl | Racemat ; |
| isopropyl | CH₂CH₂OCH₃ | 2-CH₃ | 3-F | Racemat ; |
| isopropyl | CH₂CH₂OCH₃ | 2-C=CH | 3-Cl | Racemat ; |
| isopropyl | CH₂CH₂OCN₃ | 2-Cl | 5-C=CH | Racemat ; |
| isopropyl | CH₂CH₂OCH₃ | 2-C=CH | 4-F | Racemat ; |
| isopropyl | CH₂CH₂OCH₃ | 3-OCF₃ | H | Racemat ; |
| isopropyl | CH₂CH₂OCH₃ | 2-SCH₃ | 5-Br | Racemat ; |
| isopropyl | CH₂CH₂OCH₃ | 2-OCH₃ | 3-F | Racemat ; |
| isopropyl | CH₂CH₂OCH₃ | 2-OCH₃ | 6-F | Racemat ; |
| isopropyl | CH₂CH₂OCH₃ | 2-OCH₃ | 5-F | Racemat ; |
| isopropyl | CH₂CH₂OCH₃ | 2-F | 5-OCH₃ | Racemat ; |
| isopropyl | CH₂CH₂OCH₃ | 3-Cl | H | Racemat ; |
| isopropyl | CH₂CH₂OCH₃ | 2-OCH₃ | 6-Cl | Racemat ; |
| isopropyl | CH₂CH₂OCH₃ | 2-Cl | 5-CN | Racemat ; |
| isopropyl | CH₂CH₂OCH₃ | 2-OCH₃ | 5-Cl | Racemat ; |
| isopropyl | CH₂CH₂OCH₃ | 2-F | 5-F | Racemat ; |
| isobutyl | CH₂CH₂OCH₃ | 3-F | 4-F | Racemat ; |
| 3-butyl | CH₂CH₂OCH₃ | 3-F | 4-F | Racemat ; |
| cyclopentyl | CH₂CH₂OCH₃ | 3-F | 4-F | Racemat ; |
| cyclohexyl | CH₂CH₂OCH₃ | 3-F | 4-F | Racemat ; |
| isopropyl | n-butyl | 2-F | 6-Cl | Racemat ; |
| cyclopentyl | n-butyl | 2-F | 6-Cl | Racemat ; |
| methyl | CH₂CH₂OCH₃ | 3-CN | H | Racemat ; |
| isopropyl | CH₂CH₂OH | 3-CN | H | Racemat ; |
| isopropyl | CH₂CH₂OCH₂CH₃ | 3-CN | H | Racemat ; |
| isopropyl | CH₂CH₂OCH(CH₃)₂ | 3-CN | H | Racemat ; |
| isopropyl | CH₂CH₂OCH₂CH(CH₃)₂ | 3-CN | H | Racemat ; |
| isopropyl | CH₂CH₂OC(CH₃)₃ | 3-CN | H | Racemat ; |
| isopropyl | CH₂CH₂CH₂OCH₃ | 3-CN | H | Racemat ; |
| isopropyl | CH₂C(CH₃)₂CH₂OCH₃ | 3-CN | H | Racemat ; |
| isobutyl | CH₂CH₂OCH₃ | 3-CN | H | Racemat ; |
| cyclopentyl | CH₂CH₂OH | 3-CN | H | Racemat ; |
| cyclopentyl | CH₂CH₂OCH₃ | 3-CN | H | Racemat ; |
| cyclopentyl | CH₂CH₂OCH₂CH₃ | 3-CN | H | Racemat ; |
| cyclopentyl | CH₂CH₂OCH(CH₃)₂ | 3-CN | H | Racemat ; |
| cyclopentyl | CH₂CH₂OCH(CH₃)₂ | 3-CN | H | Racemat ; |
| cyclopentyl | CH₂CH₂OC(CH₃)₃ | 3-CN | H | Racemat ; |
| cyclopentyl | CH₂C(CH₃)₂CH₂OCH₃ | 3-CN | H | Racemat ; |
| cyclohexyl | CH₂CH₂OCH₃ | 3-CN | H | Racemat ; |
| CH₂CH₂OCH₃ | CH₂CH₂OCH₃ | 3-CN | H | ; |
| cyclopentyl | CH₂CH₂OCH₃ | 2-Cl | 5-CN | Racemat ; |
| cyclopentyl | methyl | 2-CN | 3-Cl | Racemat ; |
| t-butyl | CH₂CH₂OCH₃ | 2-F | 6-F | Racemat ; |
| cyclopentyl | CH₂CH₂OCH(CH₃)₂ | 2-F | 3-F | Racemat ; |
| cyclopentyl | CH₂CH₂OH | 2-F | 3-F | Racemat ; |
| isopropyl | CH₂CH₂OH | 2-F | 3-F | Racemat ; |
| isopropyl | CH₂CH₂OCH(CH₃)₂ | 2-F | 3-F | Racemat ; |
| cycloheptyl | CH₂CH₂OCH₃ | 2-F | 5-F | Racemat ; |
| isopropyl | CH₂C(CH₃)₂OH | 2-F | 5-F | Racemat ; |
| methyl | CH₂CH₂OCH₃ | 3-CN | H (-) | ; |
| Methyl | CH₂CH₂OCH₃ | 3-CN | H | (+) ; |
| isopropyl | CH₂CH₂OCH₃ | 2-Cl | 5-CN | (+) ; |
| isopropyl | CH₂CH₂OCH₃ | 2-Cl | 5-CN | (-) ; |
| CH₂CH₂OCH₃ | CH₂CH₂OCH₃ | 2-F | 3-CF₃ | ; |
| CH₂CH₂OCH₃ | CH₂CH₂OCH₃ | 2-Cl | 5-Cl | ; |
| CH₂CH₂OCH₃ | CH₂CH₂OCH₃ | 2-F | 5-F | ; |
| CH₂CH₂CH₂OCH₃ | CH₂CH₂CH₂OCH₃ | 2-Cl | 5-Cl | ; |
| CH₂CH₂CH₂OCH₃ | CH₂CH₂CH₂OCH₃ | 2-F | 5-F | ; |
| CH₂C(CH₃)₂CH₂OCH₃ | CH₂C(CH₃)₂CH₂OCH₃ | 2-Cl | 5-Cl | ; |
| CH₂C(CH₃)₂CH₂OCH₃ | CH₂C(CH₃)₂CH₂OCH₃ | 2-F | 5-F | ; |
| methyl | CH₂CH₂OCH₃ | 2-F | 3-F | Racemat ; |
| CH₂CH₂OCH₃ | CH₂CH₂OCH₃ | 2-F | 3-F | ; |
| CH₂CH₂OCH₃ | CH₂CH₂OCH₃ | 2-Cl | H | ; |
| isopropyl | CH₂CH₂OCH₃ | 2-Cl | H | Racemat ; |
| | | | | oder |
| isopropyl | CH₂C(CH₃)₂CH₂OH | 2-Cl | H | Racemat |
und deren physiologisch unbedenklichen Salze.

9. Verbindungen nach Anspruch 8 ausgewählt aus der Grupppe
(±) Isopropyl-(2-methoxyethyl)-4-(2-fluor-phenyl)-1,4-dihydro-2,6-dimethylpyridin-3,5-dicarboxylat ;
(+) Isopropyl-(2-methoxyethyl)-4-(2-fluor-phenyl)-1,4-dihydro-2,6-dimethylpyridin-3,5-dicarboxylat;
(-) Isopropyl-(2-methoxyethyl)-4-(2-Fluor-phenyl)-1,4-dihydro-2,6-dimethylpyridin-3,5-dicarboxylat;
(±) Isopropyl-(2-methoxyethyl)-4-(2,3-difluor-phenyl)-1,4-dihydro-2,6-dimethylpyridin-3,5-dicarboxylat ;
(+) Isopropyl-(2-methoxyethyl)-4-(2,3-difluor-phenyl)-1,4-dihydro-2,6-dimethylpyridin-3,5-dicarboxylat ;
(-) Isopropyl-(2-methoxyethyl)-4-(2,3-difluor-phenyl)-1,4-dihydro-2,6-dimethylpyridin-3,5-dicarboxylat;
(±) Isopropyl-(2-methoxyethyl)-4-(2,5-difluor-phenyl)-1,4-dihydro-2,6-dimethylpyridin-3,5-dicarboxylat ;
(+) Isopropyl-(2-methoxyethyl)-4-(2,5-difluor-phenyl)-1,4-dihydro-2,6-dimethylpyridin-3,5-dicarboxylat;
(-) Isopropyl-(2-methoxyethyl)-4-(2,5-difluor-phenyl)-1,4-dihydro-2,6-dimethylpyridin-3,5-dicarboxylat ;
(±) Isopropyl-(2-methoxyethyl)-4-(2,6-difluor-phenyl)-1,4-dihydro-2,6-dimethylpyridin-3,5-dicarboxylat ;
(+) Isopropyl-(2-methoxyethyl)-4-(2,6-difluor-phenyl)-1,4-dihydro-2,6-dimethylpyridin-3,5-dicarboxylat;
(-) Isopropyl-(2-methoxyethyl)-4-(2,6-difluor-phenyl)-1,4-dihydro-2,6-dimethylpyridin-3,5-dicarboxylat;
(±) Isopropyl-(2-methoxyethyl)-4-(2,5-dichlor-phenyl)-1,4-dihydro-2,6-dimethylpyridin-3,5-dicarboxylat;
(+) Isopropyl-(2-methoxyethyl)-4-(2,5-dichlor-phenyl)-1,4-dihydro-2,6-dimethylpyridin-3,5-dicarboxylat ;
(-) Isopropyl-(2-methoxyethyl)-4-(2,5-dichlor-phenyl)-1,4-dihydro-2,6-dimethylpyridin-3,5-dicarboxylat;
(±) Isopropyl-(2-methoxyethyl)-4-(2-chlor-6-fluor-phenyl)-1,4-dihydro-2,6-dimethylpyridin-3,5-dicarboxylat;
(+) Isopropyl-(2-methoxyethyl)-4-(2-chlor-6-fluor-phenyl)-1,4-dihydro-2,6-dimethylpyridin-3,5-dicarboxylat;
(-) Isopropyl-(2-methoxyethyl)-4-(2-chlor-6-fluor-phenyl)-1,4-dihydro-2,6-dimethylpyridin-3,5-dicarboxylat;
(±) Isopropyl-(2-methoxyethyl)-4-(2-fluor-3-trifluor-methylphenyl)-1,4-dihydro-2,6-dimethylpyridin-3,5-dicarboxylat;
(+) Isopropyl-(2-methoxyethyl)-4-(2-fluor-3-trifluor-methylphenyl)-1,4-dihydro-2,6-dimethylpyridin-3,5-dicarboxylat;
(-) Isopropyl-(2-methoxyethyl)-4-(2-fluor-3-trifluor-methylphenyl)-1,4-dihydro-2,6-dimethylpyridin-3,5-dicarboxylat ;
(±) Isopropyl-(2-methoxyethyl)-4-(3-chlor-2-cyano-phenyl)-1,4-dihydro-2,6-dimethylpyridin-3,5-dicarboxylat ;
(+) Isopropyl-(2-methoxyethyl)-4-(3-chlor-2-cyano-phenyl)-1,4-dihydro-2,6-dimethylpyridin-3,5-dicarboxylat ;
(-) Isopropyl-(2-methoxyethyl)-4-(3-chlor-2-cyano-phenyl)-1,4-dihydro-2,6-dimethylpyridin-3,5-dicarboxylat ;
(±) Isopropyl-(2-methoxyethyl)-4-(2-cyano-phenyl)-1,4-dihydro-2,6-dimethylpyridin-3,5-dicarboxylat ;
(+) Isopropyl-(2-methoxyethyl)-4-(2-cyano-phenyl)-1,4-dihydro-2,6-dimethylpyridin-3,5-dicarboxylat ;
(-) Isopropyl-(2-methoxyethyl)-4-(2-cyano-phenyl)-1,4-dihydro-2,6-dimethylpyridin-3,5-dicarboxylat ;
(±) tert.Butyl-(2-methoxyethyl)-4-(3-cyano-phenyl)-1,4-dihydro-2,6-dimethylpyridin-3,5-dicarboxylat;
(+) tert.Butyl-(2-methoxyethyl)-4-(3-cyano-phenyl)-1,4-dihydro-2,6-dimethylpyridin-3,5-dicarboxylat ;
(-) tert.Butyl-(2-methoxyethyl)-4-(3-cyano-phenyl)-1,4-dihydro-2,6-dimethylpyridin-3,5-dicarboxylat;
(±) Cycloheptyl-(2-methoxyethyl)-4-(3-cyano-phenyl)-1,4-dihydro-2,6-dimethylpyridin-3,5-dicarboxylat ;
(+) Cycloheptyl-(2-methoxyethyl)-4-(3-cyano-phenyl)-1,4-dihydro-2,6-dimethylpyridin-3,5-dicarboxylat ;
(-) Cycloheptyl-(2-methoxyethyl)-4-(3-cyano-phenyl)-1,4-dihydro-2,6-dimethylpyridin-3,5-dicarboxylat ;
(±) Cyclopentyl-(2-methoxyethyl)-4-(2-chlor-3-cyano-phenyl)-1,4-dihydro-2,6-dimethylpyridin-3,5-dicarboxylat;
(+) Cyclopentyl-(2-methoxyethyl)-4-(2-chlor-3-cyano-phenyl)-1,4-dihydro-2,6-dimethylpyridin-3,5-dicarboxylat;
(-) Cyclopentyl-(2-methoxyethyl)-4-(2-chlor-3-cyano-phenyl)-1,4-dihydro-2,6-dimethylpyridin-3,5-dicarboxylat ;
(±) Cyclopentyl-(2-methoxyethyl)-4-(2-fluor-3-trifluormethyl-phenyl)-1,4-dihydro-2,6-dimethylpyridin-3,5-dicarboxylat;
(+) Cyclopentyl-(2-methoxyethyl)-4-(2-fluor-3-trifluormethyl-phenyl)-1,4-dihydro-2,6-dimethylpyridin-3,5-dicarboxylat;
(-) Cyclopentyl-(2-methoxyethyl)-4-(2-fluor-3-trifluormethyl-phenyl)-1,4-dihydro-2,6-dimethylpyridin-3,5-dicarboxylat;
(±) (2-Methoxyethyl)-(methyl)-4-(2-fluor-3-trifluormethyl-phenyl)-1,4-dihydro-2,6-dimethylpyridin-3,5-dicarboxylat ;
(+) (2-Methoxyethyl)-(methyl)-4-(2-fluor-3-trifluormethyl-phenyl)-1,4-dihydro-2,6-dimethylpyridin-3,5-dicarboxylat;
(-) (2-Methoxyethyl)-(methyl)-4-(2-fluor-3-trifluormethyl-phenyl)-1,4-dihydro-2,6-dimethylpyridin-3,5-dicarboxylat;
(±) Cyclopentyl-(2-methoxyethyl)-4-(2-cyano-3-chlor-phenyl)-1,4-dihydro-2,6-dimethylpyridin-3,5-dicarboxylat;
(+) Cyclopentyl-(2-methoxyethyl)-4-(2-cyano-3-chlor-phenyl)-1,4-dihydro-2,6-dimethylpyridin-3,5-dicarboxylat; und
(-) Cyclopentyl-(2-methoxyethyl)-4-(2-cyano-3-chlor-phenyl)-1,4-dihydro-2,6-dimethylpyridin-3,5-dicarboxylat.

## Claims

1. Use of compounds of the general formula 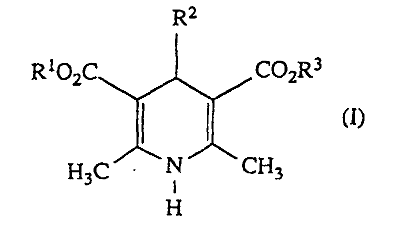 in which
R¹ and R³ are identical or different and represent straight-chain or branched alkyl having up to 8 carbon atoms, which is optionally substituted by straight-chain or branched alkoxy having up to 6 carbon atoms or hydroxyl, or
represent cycloalkyl having 3 to 7 carbon atoms,
and R² represents the radical 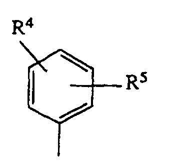 in which
R⁴ and R⁵ are identical or different and represent halogen, cyano, ethinyl, trifluoromethoxy, methyl, methylthio, trifluoromethyl or straight-chain or branched alkoxy having up to 4 carbon atoms,
or
R⁴ or R⁵ represents hydrogen
for the production of a medicament for the treatment of central degenerative disorders or brain power disorders.

2. Use according to Claim 1, where
R¹ and R³ are identical or different and represent straight-chain or branched alkyl having up to 8 carbon atoms, which is optionally substituted by straight-chain or branched alkoxy having up to 5 carbon atoms or hydroxyl, or
represent cyclopropyl, cyclopentyl, cyclohexyl or cycloheptyl,
and R² represents the radical 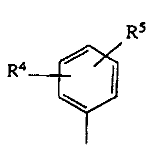 in which
R⁴ and R⁵ are identical or different and represent fluorine, chlorine, bromine, cyano, ethinyl, trifluoromethoxy, methyl, methylthio, trifluoromethyl or straight-chain or branched alkoxy having up to 3 carbon atoms,
or
R⁴ or R⁵ represents hydrogen.

3. Use according to Claim 1, where
R¹ and R³ are identical or different and represent straight-chain or branched alkyl having up to 8 carbon atoms, which is optionally substituted by methoxy or hydroxyl, or
represent cyclopentyl, cyclopropyl, cyclohexyl or cycloheptyl,
and R² represents the radical 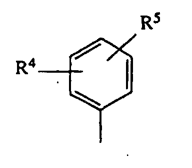 in which
R⁴ and R⁵ are identical or different and represent fluorine, chlorine, bromine, cyano, ethinyl, trifluoromethoxy, methyl, methylthio, trifluoromethyl or straight-chain or branched alkoxy having up to 3 carbon atoms,
or
R⁴ or R⁵ represents hydrogen.

4. Use according to Claim 1, where
R³ represents the radical -(CH₂)ₙ-OR⁶, in which n represents a number from 2 to 4 and R⁶ represents hydrogen or alkyl having 1 to 4 C atoms and
R¹ is identical to or different from R³ and represents cyclopropyl, cyclopentyl, cyclohexyl or cycloheptyl or represents alkyl having up to 8 C atoms, which is optionally substituted by hydroxyl or alkoxy having 1 to 4 C atoms,
and
R² has the meaning indicated in Claim 3.

5. Use according to Claim 1, where
R¹ and R³ have the meaning indicated in Claim 4, and
R² represents a phenyl radical which is substituted in the 2- and 3-position by identical or different fluorine, chlorine, cyano or CF₃ substituents, 2,3-dichlorophenyl being excluded.

6. Use according to Claim 4, in which
R³ represents the radical -CH₂-CH₂-OCH₃, and R¹ and R² have the meaning indicated in Claim 4.

7. Process for the preparation of phenyl-substituted 1,4-dihydropyridines according to Claim 1, **characterized in that**
[A] aldehydes of the general formula (II)
R²-CHO (II)
in which
R² has the meaning indicated in Claim 1,
are reacted first with acetoacetic esters of the general formula (III)
H₃C-CO-CH₂-CO₂R¹ (III)
in which
R¹ has the meaning indicated in Claim 1,
if appropriate with isolation of the corresponding ylidene compounds of the general formula (IV) 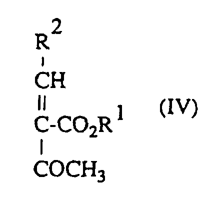 and these are then reacted either with compounds of the general formula (V)
CH₃-CO-CH₂-CO₂R³ (V)
in which
R³ has the meaning indicated in Claim 1,
in inert solvents, in the presence of ammonia or ammonium salts,
or directly with enamino derivatives of the general formula (VI) 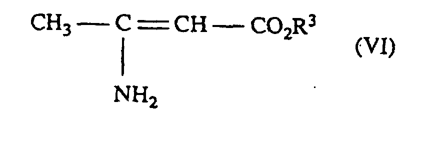 in which
R³ has the meaning indicated above,
or
[B] the aldehydes of the general formula (II) are reacted first with the compounds of the general formula (V), if appropriate with isolation of the ylidene compounds of the general formula (VII) 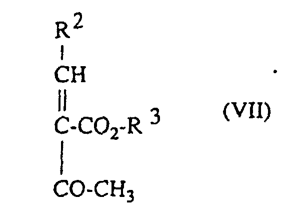 in which
R² and R³ have the meaning indicated above,
and these are reacted in a next step with the compounds of the general formula (III) in inert solvents, in the presence of ammonia or ammonium salts or directly with enaminocarboxylic acid derivatives of the general formula (VIII) 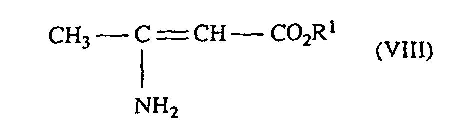 in which
R¹ has the meaning indicated above,
or
[C] compounds of the general formula (IX) 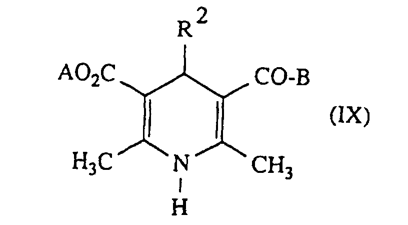 in which
R² has the meaning indicated above,
A has the meaning of R¹ or R³ indicated above
and
B together with the -CO- group forms a reactive carboxylic acid derivative
are reacted in inert solvents, in the presence of a base,
with compounds of the general formula (X)
R⁶-OH (X)
in which
R⁶ has the meaning of R¹ or R³ indicated above,
and, in the case of the pure ester enantiomers, the enantiomerically pure carboxylic acids are reacted, if appropriate first via the stage of a reactive acid derivative, with the corresponding alcohols.

8. Compounds of the general formula (I) 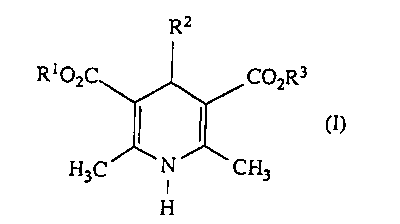 in which
R² represents the radical 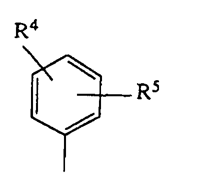 selected from a group of compounds in which the substituents R¹, R³, R⁴ and R⁵ have the meanings recited in the table which follows:
| **R**^{**1**} | **R**^{**3**} | **R**^{**4**} | **R**^{**5**} | **Racemate, (+)-,(-)-enantiomer** |
|---|---|---|---|---|
| isopropyl | CH₂CH₂OCH₃ | 2-F | H | Racemate |
| cycloheptyl | CH₂CH₂OCH₃ | 3-CN | H | (-) ; |
| isopropyl | CH₂CH₂OCH₃ | 2-Cl | 6-F | Racemate |
| isopropyl | CH₂CH₂OCH₃ | 2-Cl | 6-F | (+) ; |
| isopropyl | CH₂CH₂OCH₃ | 2-Cl | 6-F | (-) ; |
| isopropyl | CH₂CH₂OCH₃ | 2-F | 6-F | Racemate ; |
| isopropyl | CH₂CH₂OCH₃ | 2-F | 6-F | (+) ; |
| isopropyl | CH₂CH₂OCH₃ | 2-F | 6-F | (-) ; |
| isopropyl | CH₂CH₂OCH₃ | 2-F | 3-F | Racemate |
| isopropyl | CH₂CH₂OCH₃ | 2-F | 3-F | (+) ; |
| isopropyl | CH₂CH₂OCH₃ | 2-F | 3-F | (-) ; |
| isopropyl | CH₂CH₂OCH₃ | 2-Cl | 5-Cl | Racemate |
| isopropyl | CH₂CH₂OCH₃ | 2-Cl | 5-Cl | (+) ; |
| isopropyl | CH₂CH₂OCH₃ | 2-Cl | 5-Cl | (-) ; |
| isopropyl | CH₂CH₂OCH₃ | 2-CN | 3-Cl | Racemate |
| isopropyl | CH₂CH₂OCH₃ | 2-CN | 3-Cl | (+) ; |
| isopropyl | CH₂CH₂OCH₃ | 2-CN | 3-Cl | (-) ; |
| isopropyl | CH₂CH₂OCH₃ | 3-F | 4-F | Racemate |
| isopropyl | CH₂CH₂OCH₃ | 3-F | 4-F | (+) ; |
| isopropyl | CH₂CH₂OCH₃ | 3-F | 4-F | (-) ; |
| isopropyl | CH₂CH₂OCH₃ | 2-F | 5-F | Racemate ; |
| isopropyl | CH₂CH₂OCH₃ | 2-F | 5-F | (+) ; |
| isopropyl | CH₂CH₂OCH₃ | 2-F | 5-F | (-) ; |
| isopropyl | CH₂CH₂OCH₃ | 2-CN | H | Racemate ; |
| isopropyl | CH₂CH₂OCH₃ | 2-CN | H | (+) ; |
| isopropyl | CH₂CH₂OCH₃ | 2-CN | H | (-) ; |
| isopropyl | CH₂CH₂OCH₃ | 2-F | 3-CF₃ | Racemate ; |
| isopropyl | CH₂CH₂OCH₃ | 2-F | 3-CF₃ | (+) ; |
| isopropyl | CH₂CH₂OCH₃ | 2-F | 3-CF₃ | (-) ; |
| cyclopentyl | CH₂CH₂OCH₃ | 2-Cl | 3-CN | Racemate ; |
| cyclopentyl | CH₂CH₂OCH₃ | 2-Cl | 3-CN | (+) ; |
| cyclopentyl | CH₂CH₂OCH₃ | 2-Cl | 3-CN | (-) ; |
| t-butyl | CH₂CH₂OCH₃ | 3-CN | H | Racemate |
| t-butyl | CH₂CH₂OCH₃ | 3-CN | H | (+) ; |
| t-butyl | CH₂CH₂OCH₃ | 3-CN | H | (-) ; |
| cyclopentyl | CH₂CH₂OCH₃ | 2-F | 3-CF₃ | Racemate ; |
| cyclopentyl | CH₂CH₂OCH₃ | 2-F | 3-CF₃ | (+) ; |
| cyclopentyl | CH₂CH₂OCH₃ | 2-F | 3-CF₃ | (-) ; |
| methyl | CH₂CH₂OCH₃ | 2-F | 3-CF₃ | Racemate ; |
| methyl | CH₂CH₂OCH₃ | 2-F | 3-CF₃ | (+) ; |
| methyl | CH₂CH₂OCH₃ | 2-F | 3-CF₃ | (-) ; |
| cycloheptyl | CH₂CH₂OCH₃ | 3-CN | H | Racemate ; |
| cycloheptyl | CH₂CH₂OCH₃ | 3-CN | H | (+) ; |
| isopropyl | CH₂CH₂OCH₃ | 2-F | H | (+) ; |
| isopropyl | CH₂CH₂OCH₃ | 2-F | H | (-) ; |
| cyclopentyl | CH₂CH₂OCH₃ | 2-CN | 3-Cl | Racemate ; |
| cyclopentyl | CH₂CH₂OCH₃ | 2-CN | 3-Cl | (+) ; |
| cyclopentyl | CH₂CH₂OCH₃ | 2-CN | 3-Cl | (-) ; |
| isopropyl | CH₂CH₂OCH₃ | 2-Cl | 5-CN | Racemate ; |
| cycloheptyl | CH₂CH₂OCH₃ | 3-CN | H | (-) ; |
| isopropyl | CH₂CH₂OCH₃ | 2-F | 4-F | Racemate ; |
| isopropyl | CH₂CH₂OCH₃ | 3-F | H | Racemate ; |
| isopropyl | CH₂CH₂OCH₃ | 2-Cl | 4-Cl | Racemate ; |
| isopropyl | CH₂CH₂OCH₃ | 3-Cl | 4-Cl | Racemate ; |
| isopropyl | CH₂CH₂OCH₃ | 3-Cl | 5-Cl | Racemate ; |
| isopropyl | CH₂CH₂OCH₃ | 2-Cl | 6-Cl | Racemate ; |
| isopropyl | CH₂CH₂OCH₃ | 2-F | 5-Cl | Racemate ; |
| isopropyl | CH₂CH₂OCH₃ | 3-Cl | 4-Cl | Racemate ; |
| isopropyl | CH₂CH₂OCH₃ | 2-CH₃ | 3-F | Racemate ; |
| isopropyl | CH₂CH₂OCH₃ | 2-C≡CH | 3-Cl | Racemate ; |
| isopropyl | CH₂CH₂OCH₃ | 2-Cl | 5-C≡CH | Racemate ; |
| isopropyl | CH₂CH₂OCH₃ | 2-C≡CH | 4-F | Racemate ; |
| isopropyl | CH₂CH₂OCH₃ | 3-OCF₃ | H | Racemate ; |
| isopropyl | CH₂CH₂OCH₃ | 2-SCH₃ | 5-Br | Racemate ; |
| isopropyl | CH₂CH₂OCH₃ | 2-OCH₃ | 3-F | Racemate ; |
| isopropyl | CH₂CH₂OCH₃ | 2-OCH₃ | 6-F | Racemate ; |
| isopropyl | CH₂CH₂OCH₃ | 2-OCH₃ | 5-F | Racemate ; |
| isopropyl | CH₂CH₂OCH₃ | 2-F | 5-OCH₃ | Racemate ; |
| isopropyl | CH₂CH₂OCH₃ | 3-Cl | H | Racemate ; |
| isopropyl | CH₂CH₂OCH₃ | 2-OCH₃ | 6-Cl | Racemate ; |
| isopropyl | CH₂CH₂OCH₃ | 2-Cl | 5-CN | Racemate ; |
| isopropyl | CH₂CH₂OCH₃ | 2-OCH₃ | 5-Cl | Racemate ; |
| isopropyl | CH₂CH₂OCH₃ | 2-F | 5-F | Racemate ; |
| isopropyl | CH₂CH₂OCH₃ | 3-F | 4-F | Racemate ; |
| isopropyl | CH₂CH₂OCH₃ | 3-F | 4-F | Racemate ; |
| 3-butyl | CH₂CH₂OCH₃ | 3-F | 4-F | Racemate ; |
| cyclopentyl | CH₂CH₂OCH₃ | 3-F | 4-F | Racemate ; |
| cyclohexyl | CH₂CH₂OCH₃ | 3-F | 4-F | Racemate ; |
| isopropyl | n-butyl | 2-F | 6-Cl | Racemate ; |
| cyclopentyl | n-butyl | 2-F | 6-Cl | Racemate ; |
| methyl | CH₂CH₂OCH₃ | 3-CN | H | Racemate ; |
| isopropyl | CH₂CH₂OH | 3-CN | H | Racemate ; |
| isopropyl | CH₂CH₂OCH₂CH₃ | 3-CN | H | Racemate ; |
| isopropyl | CH₂CH₂OCH(CH₃)₂ | 3-CN | H | Racemate ; |
| isopropyl | CH₂CH₂OCH₂CH(CH₃)₂ | 3-CN | H | Racemate ; |
| isopropyl | CH₂CH₂OC(CH₃)₃ | 3-CN | H | Racemate ; |
| isopropyl | CH₂CH₂CH₂OCH₃ | 3-CN | H | Racemate ; |
| isopropyl | CH₂C(CH₃)₂CH₂OCH₃ | 3-CN | H | Racemate ; |
| isobutyl | CH₂CH₂OCH₃ | 3-CN | H | Racemate ; |
| cyclopentyl | CH₂CH₂OH | 3-CN | H | Racemate ; |
| cyclopentyl | CH₂CH₂OCH₃ | 3-CN | H | Racemate ; |
| cyclopentyl | CH₂CH₂OCH₂CH₃ | 3-CN | H | Racemate ; |
| cyclopentyl | CH₂CH₂OCH(CH₃)₂ | 3-CN | H | Racemate ; |
| cyclopentyl | CH₂CH₂OCH(CH₃)₂ | 3-CN | H | Racemate ; |
| cyclopentyl | CH₂CH₂OC(CH₃)₃ | 3-CN | H | Racemate ; |
| cyclopentyl | CH₂C(CH₃)₂CH₂OCH₃ | 3-CN | H | Racemate ; |
| cyclohexyl | CH₂CH₂OCH₃ | 3-CN | H | Racemate |
| CH₂CH₂OCH₃ | CH₂CH₂OCH₃ | 3-CN | H | ; |
| cyclopentyl | CH₂CH₂OCH₃ | 2-Cl | 5-CN | Racemate ; |
| cyclopentyl | methyl | 2-CN | 3-C1 | Racemate ; |
| t-butyl | CH₂CH₂OCH₃ | 2-F | 6-F | Racemate ; |
| cyclopentyl | CH₂CH₂OCH(CH₃)₂ | 2-F | 3-F | Racemate ; |
| cyclopentyl | CH₂CH₂OH | 2-F | 3-F | Racemate ; |
| isopropyl | CH₂CH₂OH | 2-F | 3-F | Racemate ; |
| isopropyl | CH₂CH₂OCH(CH₃)₂ | 2-F | 3-F | Racemate ; |
| cyclopentyl | CH₂CH₂OCH₃ | 2-F | 5-F | Racemate ; |
| isopropyl | CH₂C(CH₃)₂OH | 2-F | 5-F | Racemate ; |
| methyl | CH₂CH₂OCH₃ | 3-CN | H | (-) ; |
| methyl | CH₂CH₂OCH₃ | 3-CN | H | (+) ; |
| isopropyl | CH₂CH₂OCH₃ | 2-Cl | 5-CN | (+) ; |
| isopropyl | CH₂CH₂OCH₃ | 2-Cl | 5-CN | (-) ; |
| CH₂CH₂OCH₃ | CH₂CH₂OCH₃ | 2-F | 3-CF₃ | ; |
| CH₂CH₂OCH₃ | CH₂CH₂OCH₃ | 2-Cl | 5-Cl | ; |
| CH₂CH₂OCH₃ | CH₂CH₂OCH₃ | 2-F | 5-F | ; |
| CH₂CH₂CH₂OCH₃ | CH₂CH₂CH₂OCH₃ | 2-Cl | 5-Cl | ; |
| CH₂CH₂CH₂OCH₃ | CH₂CH₂CH₂OCH₃ | 2-F | 5-F | ; |
| CH₂C(CH₃)₂CH₂OCH₃ | CH₂C(CH₃)₂CH₂OCH₃ | 2-Cl | 5-Cl | ; |
| CH₂C(CH₃)₂CH₂OCH₃ | CH₂C(CH₃)₂CH₂OCH₃ | 2-F | 5-F | ; |
| methyl | CH₂CH₂OCH₃ | 2-F | 3-F | Racemate ; |
| CH₂CH₂OCH₃ | CH₂CH₂OCH₃ | 2-F | 3-F | ; |
| CH₂CH₂OCH₃ | CH₂CH₂OCH₃ | 2-Cl | H | ; |
| isopropyl | CH₂CH₂OCH₃ | 2-Cl | H | Racemate ; |
| | | | | or |
| isopropyl | CH₂C(CH₃)₂CH₂OH | 2-Cl | H | Racemate |
and their physiologically acceptable salts.

9. Compounds according to Claim 8 selected from the group consisting of
(±) isopropyl 2-methoxyethyl 4-(2-fluorophenyl)-1,4-dihydro-2,6-dimethylpyridine-3,5-dicarboxylate;
(+) isopropyl 2-methoxyethyl 4-(2-fluorophenyl)-1,4-dihydro-2,6-dimethylpyridine-3,5-dicarboxylate;
(-) isopropyl 2-methoxyethyl 4-(2-fluorophenyl)-1,4-dihydro-2,6-dimethylpyridine-3,5-dicarboxylate;
(±) isopropyl 2-methoxyethyl 4-(2,3-difluorophenyl)-1,4-dihydro-2,6-dimethylpyridine-3,5-dicarboxylate;
(+) isopropyl 2-methoxyethyl 4-(2,3-difluorophenyl)-1,4-dihydro-2,6-dimethylpyridine-3,5-dicarboxylate;
(-) isopropyl 2-methoxyethyl 4-(2,3-difluorophenyl)-1,4-dihydro-2,6-dimethylpyridine-3,5-dicarboxylate;
(±) isopropyl 2-methoxyethyl 4-(2,5-difluorophenyl)-1,4-dihydro-2,6-dimethylpyridine-3,5-dicarboxylate;
(+) isopropyl 2-methoxyethyl 4-(2,5-difluorophenyl)-1,4-dihydro-2,6-dimethylpyridine-3,5-dicarboxylate;
(-) isopropyl 2-methoxyethyl 4-(2,5-difluorophenyl)-1,4-dihydro-2,6-dimethylpyridine-3,5-dicarboxylate;
(±) isopropyl 2-methoxyethyl 4-(2,6-difluorophenyl)-1,4-dihydro-2,6-dimethylpyridine-3,5-dicarboxylate;
(+) isopropyl 2-methoxyethyl 4-(2,6-difluorophenyl)-1,4-dihydro-2,6-dimethylpyridine-3,5-dicarboxylate;
(-) isopropyl 2-methoxyethyl 4-(2,6-difluorophenyl)-1,4-dihydro-2,6-dimethylpyridine-3,5-dicarboxylate;
(±) isopropyl 2-methoxyethyl 4-(2,5-dichlorophenyl)-1,4-dihydro-2,6-dimethylpyridine-3,5-dicarboxylate;
(+) isopropyl 2-methoxyethyl 4-(2,5-dichlorophenyl)-1,4-dihydro-2,6-dimethylpyridine-3,5-dicarboxylate;
(-) isopropyl 2-methoxyethyl 4-(2,5-dichlorophenyl)-1,4-dihydro-2,6-dimethylpyridine-3,5-dicarboxylate;
(±) isopropyl 2-methoxyethyl 4-(2-chloro-6-fluorophenyl)-1,4-dihydro-2,6-dimethylpyridine-3,5-dicarboxylate;
(+) isopropyl 2-methoxyethyl 4-(2-chloro-6-fluorophenyl)-1,4-dihydro-2,6-dimethylpyridine-3,5-dicarboxylate;
(-) isopropyl 2-methoxyethyl 4-(2-chloro-6-fluorophenyl)-1,4-dihydro-2,6-dimethylpyridine-3,5-dicarboxylate;
(±) isopropyl 2-methoxyethyl 4-(2-fluoro-3-trifluoromethylphenyl)-1,4-dihydro-2,6-dimethylpyridine-3,5-dicarboxylate;
(+) isopropyl 2-methoxyethyl 4-(2-fluoro-3-trifluoromethylphenyl)-1,4-dihydro-2,6-dimethylpyridine-3,5-dicarboxylate;
(-) isopropyl 2-methoxyethyl 4-(2-fluoro-3-trifluoromethylphenyl)-1,4-dihydro-2,6-dimethylpyridine-3,5-dicarboxylate;
(±) isopropyl 2-methoxyethyl 4-(3-chloro-2-cyanophenyl)-1,4-dihydro-2,6-dimethylpyridine-3,5-dicarboxylate;
(+) isopropyl 2-methoxyethyl 4-(3-chloro-2-cyanophenyl)-1,4-dihydro-2,6-dimethylpyridine-3,5-dicarboxylate;
(-) isopropyl 2-methoxyethyl 4-(3-chloro-2-cyanophenyl)-1,4-dihydro-2,6-dimethylpyridine-3,5-dicarboxylate;
(±) isopropyl 2-methoxyethyl 4-(2-cyanophenyl)-1,4-dihydro-2,6-dimethylpyridine-3,5-dicarboxylate;
(+) isopropyl 2-methoxyethyl 4-(2-cyanophenyl)-1,4-dihydro-2,6-dimethylpyridine-3,5-dicarboxylate;
(-) isopropyl 2-methoxyethyl 4-(2-cyanophenyl)-1,4-dihydro-2,6-dimethylpyridine-3,5-dicarboxylate;
(±) tert-butyl 2-methoxyethyl 4-(3-cyanophenyl)-1,4-dihydro-2,6-dimethylpyridine-3,5-dicarboxylate;
(+) tert-butyl 2-methoxyethyl 4-(3-cyanophenyl)-1,4-dihydro-2,6-dimethylpyridine-3,5-dicarboxylate;
(-) tert-butyl 2-methoxyethyl 4-(3-cyanophenyl)-1,4-dihydro-2,6-dimethylpyridine-3,5-dicarboxylate;
(±) cycloheptyl 2-methoxyethyl 4-(3-cyanophenyl)-1,4-dihydro-2,6-dimethylpyridine-3,5-dicarboxylate;
(+) cycloheptyl 2-methoxyethyl 4-(3-cyanophenyl)-1,4-dihydro-2,6-dimethylpyridine-3,5-dicarboxylate;
(-) cycloheptyl 2-methoxyethyl 4-(3-cyanophenyl)-1,4-dihydro-2,6-dimethylpyridine-3,5-dicarboxylate;
(±) cyclopentyl 2-methoxyethyl 4-(2-chloro-3-cyanophenyl)-1,4-dihydro-2,6-dimethylpyridine-3,5-dicarboxylate;
(+) cyclopentyl 2-methoxyethyl 4-(2-chloro-3-cyanophenyl) -1,4-dihydro-2,6-dimethylpyridine-3,5-dicarboxylate;
(-) cyclopentyl 2-methoxyethyl 4-(2-chloro-3-cyanophenyl)-1,4-dihydro-2,6-dimethylpyridine-3,5-dicarboxylate;
(±) cyclopentyl 2-methoxyethyl 4-(2-fluoro-3-trifluoromethylphenyl)-1,4-dihydro-2,6-dimethylpyridine-3,5-dicarboxylate;
(+) cyclopentyl 2-methoxyethyl 4-(2-fluoro-3-trifluoromethylphenyl)-1,4-dihydro-2,6-dimethylpyridine-3,5-dicarboxylate;
(-) cyclopentyl 2-methoxyethyl 4-(2-fluoro-3-trifluoromethylphenyl)-1,4-dihydro-2,6-dimethylpyridine-3,5-dicarboxylate;
(±) 2-methoxyethyl methyl 4-(2-fluoro-3-trifluoromethylphenyl)-1,4-dihydro-2,6-dimethylpyridine-3,5-dicarboxylate;
(+) 2-methoxyethyl methyl 4-(2-fluoro-3-trifluoromethylphenyl)-1,4-dihydro-2,6-dimethylpyridine-3,5-dicarboxylate;
(-) 2-methoxyethyl methyl 4-(2-fluoro-3-trifluoromethylphenyl)-1,4-dihydro-2,6-dimethylpyridine-3,5-dicarboxylate;
(±) cyclopentyl 2-methoxyethyl 4-(2-cyano-3-chlorophenyl)-1,4-dihydro-2,6-dimethylpyridine-3,5-dicarboxylate;
(+) cyclopentyl 2-methoxyethyl 4-(2-cyano-3-chlorophenyl)-1,4-dihydro-2,6-dimethylpyridine-3,5-dicarboxylate; and
(-) cyclopentyl 2-methoxyethyl 4-(2-cyano-3-chlorophenyl)-1,4-dihydro-2,6-dimethylpyridine-3,5-dicarboxylate.

## Revendications

1. Utilisation de composés de formule générale 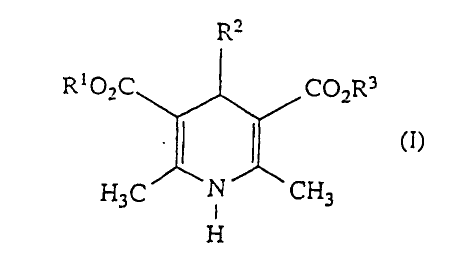 dans laquelle
R¹ et R³ sont identiques ou différents et représentent un reste alkyle linéaire ou ramifié ayant jusqu'à 8 atomes de carbone, qui est éventuellement substitué par un radical alkoxy linéaire ou ramifié ayant jusqu'à 6 atomes de carbone ou par un radical hydroxy, ou bien
un reste cycloalkyle ayant 3 à 7 atomes de carbone,
et R² représente le reste 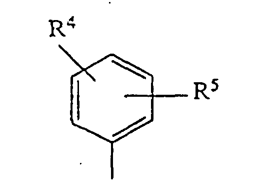 dans lequel
R⁴ et R⁵ sont identiques ou différents et représentent un halogène, un reste cyano, éthynyle, trifluorométhoxy, méthyle, méthylthio, trifluorométhyle ou un reste alkoxy linéaire ou ramifié ayant jusqu'à 4 atomes de carbone,
ou bien
R⁴ ou R⁵ représente l'hydrogène,
pour la préparation d'un médicament destiné au traitement de maladies dégénératives du système nerveux central ou de troubles des capacités mentales.

2. Utilisation suivant la revendication 1, dans laquelle
R¹ et R³ sont identiques ou différents et représentent un reste alkyle linéaire ou ramifié ayant jusqu'à 8 atomes de carbone, qui est éventuellement substitué par un radical alkoxy linéaire ou ramifié ayant jusqu'à 5 atomes de carbone ou par un radical hydroxy, ou bien
un reste cyclopropyle, cyclopentyle, cyclohexyle ou cycloheptyle,
et R² représente le reste 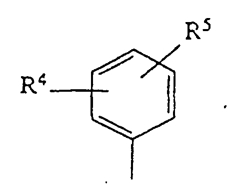 dans lequel
R⁴ et R⁵ sont identiques ou différents et représentent le fluor, le chlore, le brome, un reste cyano, éthynyle, trifluorométhoxy, méthyle, méthylthio, trifluorométhyle ou un reste alkoxy linéaire ou ramifié ayant jusqu'à 3 atomes de carbone,
ou bien
R⁴ ou R⁵ représente l'hydrogène.

3. Utilisation suivant la revendication 1, dans laquelle
R¹ et R³ sont identiques ou différents et représentent un reste alkyle linéaire ou ramifié ayant jusqu'à 8 atomes de carbone, qui est éventuellement substitué par un radical méthoxy ou hydroxy, ou bien
un reste cyclopentyle, cyclopropyle, cyclohexyle ou cycloheptyle,
et R² représente le reste 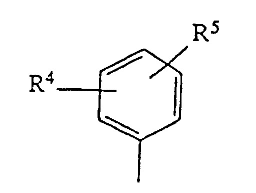 dans lequel
R⁴ et R⁵ sont identiques ou différents et représentent le fluor, le chlore, le brome, un reste cyano, éthynyle, trifluorométhoxy, méthyle, méthylthio, trifluorométhyle ou un reste alkoxy linéaire ou ramifié ayant jusqu'à 3 atomes de carbone,
ou bien
R⁴ et R⁵ représentent l'hydrogène.

4. Utilisation suivant la revendication 1, dans laquelle
R³ représente le reste -(CH2)ₙ-OR⁶, dans lequel n est un nombre de 2 à 4 et R⁶ est l'hydogène ou un radical alkyle ayant 1 à 4 atomes de carbone
et
R¹ et R³ sont identiques ou différents et représentent un reste cyclopropyle, cyclopentyle, cyclohexyle ou cycloheptyle ou un reste alkyle ayant jusqu'à 8 atomes de carbone, qui est éventuellement substitué par un radical hydroxy ou alkoxy ayant 1 à 4 atomes de carbone,
et
R² a la définition indiquée dans la revendication 3.

5. Utilisation suivant la revendication 1, dans laquelle
R¹ et R³ ont la définition indiquée dans la revendication 4, et
R² est un reste phényle qui porte en positions 2 et 3 des substituants fluoro, chloro, cyano ou CF₃ identiques ou différents, le reste 2,3-dichlorophényle étant exclu.

6. Utilisation suivant la revendication 4, dans laquelle
R³ représente le reste -CH₂-CH₂-OCH₃ et R¹ et R² ont la définition indiquée dans la revendication 4.

7. Procédé de production de 1,4-dihydropyridines à substituant phényle suivant la revendication 8, **caractérisé en ce que**
[A] on fait réagir des aldéhydes de formule générale (II)
R²-CHO (II)
dans laquelle
R² a la définition indiquée dans la revendication 8,
tout d'abord avec des esters d'acide acétylacétique de formule générale (III)
H₃C-CO-CH₂-CO₂R¹ (III)
dans laquelle
R¹ a la définition indiquée dans la revendication 8,
en isolant éventuellement les composés ylidéniques correspondants de formule générale (IV) 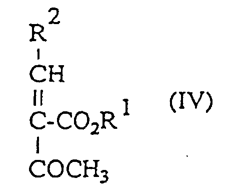
et ensuite, soit avec des composés de formule générale (V)
CH₃-CO-CH₂-CO₂R³ (V)
dans laquelle
R³ a la définition indiquée dans la revendication 8,
dans des solvants inertes, en présence d'ammoniac ou de sels d'ammonium,
soit directement avec des dérivés énamino de formule générale (VI) 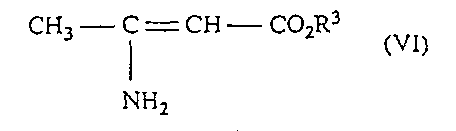 dans laquelle
R³ a la définition indiquée ci-dessus,
ou bien
[B] on fait réagir les aldéhydes de formule générale (II) d'abord avec des composés de formule générale (V), en isolant éventuellement les composés ylidéniques de formule générale (VII) 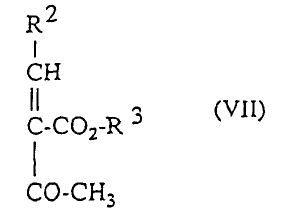 dans laquelle
R² et R³ ont la définition indiquée ci-dessus,
et dans une étape subséquente, avec les composés de formule générale (III) dans des solvants inertes, en présence d'ammoniac ou de sels d'ammonium, ou directement avec des dérivés d'acides énaminocarboxyliques de formule générale (VIII) 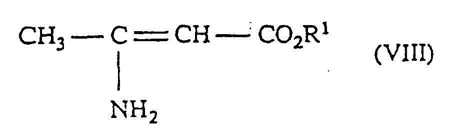 dans laquelle
R¹ a la définition indiquée ci-dessus,
ou bien
[C] on fait réagir des composés de formule générale (IX) 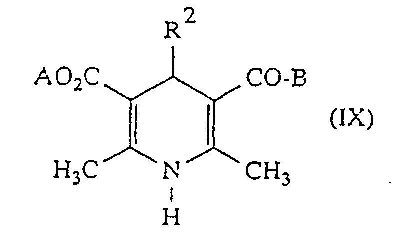
dans laquelle
R² a la définition indiquée ci-dessus,
A a la définition indiquée ci-dessus pour R¹ ou R³
et
B forme conjointement avec le groupe -CO- un dérivé d'acide carboxylique réactif,
dans des solvants inertes, en présence d'une base, avec des composés de formule générale (X)
R⁶-OH (X)
dans laquelle
R⁶ a la définition indiquée ci-dessus pour R¹ ou R³,
et dans le cas des esters énantiomères purs, on fait réagir les acides carboxyliques énantiomères purs avec les alcools correspondants, en passant éventuellement tout d'abord par le stade d'un dérivé d'acide réactif.

8. Composés de formule générale (I) 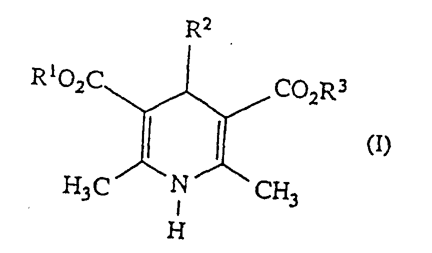 dans laquelle
R² représente le reste 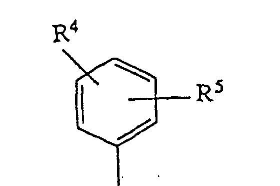 choisi dans le groupe de composés dans lesquels les substituants R¹, R³, R⁴ et R⁵ ont les définitions énumérées dans le tableau suivant :
| **R**^{**1**} | **R**^{**3**} | **R**^{**4**} | **R**^{**5**} | Racémate, (+)-, (-)-énantiomère |
|---|---|---|---|---|
| isopropyle | CH₂CH₂OCH₃ | 2-F | H | Racémate ; |
| cycloheptyle | CH₂CH₂OCH₃ | 3-CN | H | (-) ; |
| isopropyle | CH₂CH₂OCH₃ | 2-Cl | 6-F | Racémate ; |
| isopropyle | CH₂CH₂OCH₃ | 2-Cl | 6-F | (+) ; |
| isopropyle | CH₂CH₂OCH₃ | 2-Cl | 6-F | (-) ; |
| isopropyle | CH₂CH₂OCH₃ | 2-F | 6-F | Racémate ; |
| isopropyle | CH₂CH₂OCH₃ | 2-F | 6-F | (+) ; |
| isopropyle | CH₂CH₂OCH₃ | 2-F | 6-F | (-) ; |
| isopropyle | CH₂CH₂OCH₃ | 2-F | 3-F | Racémate ; |
| isopropyle | CH₂CH₂OCH₃ | 2-F | 3-F | (+) ; |
| isopropyle | CH₂CH₂OCH₃ | 2-F | 3-F | (-) ; |
| isopropyle | CH₂CH₂OCH₃ | 2-Cl | 5-CI | Racémate ; |
| isopropyle | CH₂CH₂OCH₃ | 2-Cl | 5-Cl | (+) ; |
| isopropyle | CH₂CH₂OCH₃ | 2-Cl | 5-Cl | (-) ; |
| isopropyle | CH₂CH₂OCH₃ | 2-CN | 3-CI | Racémate ; |
| isopropyle | CH₂CH₂OCH₃ | 2-CN | 3-Cl | (+) ; |
| isopropyle | CH₂CH₂OCH₃ | 2-CN | 3-Cl | (-) ; |
| isopropyle | CH₂CH₂OCH₃ | 3-F | 4-F | Racémate ; |
| isopropyle | CH₂CH₂OCH₃ | 3-F | 4-F | (+) ; |
| isopropyle | CH₂CH₂OCH₃ | 3-F | 4-F | (-) ; |
| isopropyle | CH₂CH₂OCH₃ | 2-F | 5-F | Racémate ; |
| isopropyle | CH₂CH₂OCH₃ | 2-F | 5-F | (+) ; |
| isopropyle | CH₂CH₂OCH₃ | 2-F | 5-F | (-) ; |
| isopropyle | CH₂CH₂OCH₃ | 2-CN | H | Racémate ; |
| isopropyle | CH₂CH₂OCH₃ | 2-CN | H | (+) ; |
| isopropyle | CH₂CH₂OCH₃ | 2-CN | H | (-) ; |
| isopropyle | CH₂CH₂OCH₃ | 2-F | 3-CF₃ | Racémate ; |
| isopropyle | CH₂CH₂OCH₃ | 2-F | 3-CF₃ | (+) ; |
| isopropyle | CH₂CH₂OCH₃ | 2-F | 3-CF₃ | (-) ; |
| cyclopentyle | CH₂CH₂OCH₃ | 2-CI | 3-CN | Racémate ; |
| cyclopentyle | CH₂CH₂OCH₃ | 2-CI | 3-CN | (+) ; |
| cyclopentyle | CH₂CH₂OCH₃ | 2-CI | 3-CN | (-) ; |
| tertio-butyle | CH₂CH₂OCH₃ | 3-CN | H | Racémate ; |
| tertio-butyle | CH₂CH₂OCH₃ | 3-CN | H | (+) ; |
| tertio-butyle | CH₂CH₂OCH₃ | 3-CN | H | (-) ; |
| cyclopentyle | CH₂CH₂OCH₃ | 2-F | 3-CF₃ | Racémate ; |
| cyclopentyle | CH₂CH₂OCH₃ | 2-F | 3-CF₃ | (+) ; |
| cyclopentyle | CH₂CH₂OCH₃ | 2-F | 3-CF₃ | (-) ; |
| méthyle | CH₂CH₂OCH₃ | 2-F | 3-CF₃ | Racémate ; |
| méthyle | CH₂CH₂OCH₃ | 2-F | 3-CF₃ | (+) ; |
| méthyle | CH₂CH₂OCH₃ | 2-F | 3-CF3 | (-) ; |
| cycloheptyle | CH₂CH₂OCH₃ | 3-CN | H | Racémate ; |
| cycloheptyle | CH₂CH₂OCH₃ | 3-CN | H | (+) ; |
| isopropyle | CH₂CH₂OCH₃ | 2-F | H | (+) ; |
| isopropyle | CH₂CH₂OCH₃ | 2-F | H | (-) ; |
| cyclopentyle | CH₂CH₂OCH₃ | 2-CN | 3-Cl | Racémate ; |
| cyclopentyle | CH₂CH₂OCH₃ | 2-CN | 3-Cl | (+) ; |
| cyclopentyle | CH₂CH₂OCH₃ | 2-CN | 3-Cl | (-) ; |
| isopropyle | CH₂CH₂OCH₃ | 2-CI | 5-CN | Racémate ; |
| cycloheptyle | CH₂CH₂OCH₃ | 3-CN | H | (-) ; |
| isopropyle | CH₂CH₂OCH₃ | 2-F | 4-F | Racémate ; |
| isopropyle | CH₂CH₂OCH₃ | 3-F | H | Racémate ; |
| isopropyle | CH₂CH₂OCH₃ | 2-Cl | 4-CI | Racémate ; |
| isopropyle | CH₂CH₂OCH₃ | 3-Cl | 4-CI | Racémate ; |
| isopropyle | CH₂CH₂OCH₃ | 3-Cl | 5-Cl | Racémate ; |
| isopropyle | CH₂CH₂OCH₃ | 2-Cl | 6-Cl | Racémate ; |
| isopropyle | CH₂CH₂OCH₃ | 2-F | 5-CI | Racémate ; |
| isopropyle | CH₂CH₂OCH₃ | 3-Cl | 4-CI | Racémate ; |
| isopropyle | CH₂CH₂OCH₃ | 2-CH₃ | 3-F | Racémate ; |
| isopropyle | CH₂CH₂OCH₃ | 2-C≡CH | 3-Cl | Racémate ; |
| isopropyle | CH₂CH₂OCH₃ | 2-Cl | 5-C≡CH | Racémate ; |
| isopropyle | CH₂CH₂OCH₃ | 2-C≡CH | 4-F | Racémate ; |
| isopropyle | CH₂CH₂OCH₃ | 3-OCF₃ | H | Racémate ; |
| isopropyle | CH₂CH₂OCH₃ | 2-SCH₃ | 5-Br | Racémate ; |
| isopropyle | CH₂CH₂OCH₃ | 2-OCH₃ | 3-F | Racémate ; |
| isopropyle | CH₂CH₂OCH₃ | 2-OCH₃ | 6-F | Racémate ; |
| isopropyle | CH₂CH₂OCH₃ | 2-OCH₃ | 5-F | Racémate ; |
| isopropyle | CH₂CH₂OCH₃ | 2-F | 5-OCH₃ | Racémate ; |
| isopropyle | CH₂CH₂OCH₃ | 3-CI | H | Racémate ; |
| isopropyle | CH₂CH₂OCH₃ | 2-OCH₃ | 6-CI | Racémate ; |
| isopropyle | CH₃CH₂OCH₃ | 2-Cl | 5-CN | Racémate ; |
| isopropyle | CH₂CH₂OCH₃ | 2-OCH₃ | 5-CI | Racémate ; |
| isopropyle | CH₂CH₂OCH₃ | 2-F | 5-F | Racémate ; |
| isobutyle | CH₂CH₂OCH₃ | 3-F | 4-F | Racémate ; |
| 3-butyle | CH₂CH₂OCH₃ | 3-F | 4-F | Racémate ; |
| cyclopentyle | CH₂CH₂OCH₃ | 3-F | 4-F | Racémate ; |
| cyclohexyle | CH₂CH₂OCH₃ | 3-F | 4-F | Racemate ; |
| isopropyle | n-butyle | 2-F | 6-Cl | Racémate ; |
| cyclopentyle | n-butyle | 2-F | 6-CI | Racémate ; |
| méthyle | CH₂CH₂OCH₃ | 3-CN | H | Racémate ; |
| isopropyle | CH₂CH₂OH | 3-CN | H | Racémate ; |
| isopropyle | CH₂CH₂OCH₂CH₃ | 3-CN | H | Racémate ; |
| isopropyle | CH₂CH₂OCH(CH₃)₂ | 3-CN | H | Racémate ; |
| isopropyle | CH₂CH₂OCH₂CH(CH₃)₂ | 3-CN | H | Racémate ; |
| isopropyle | CH₂CH₂OC(CH₃)₃ | 3-CN | H | Racémate ; |
| isopropyle | CH₂CH₂CH₂OCH₃ | 3-CN | H | Racémate ; |
| isopropyle | CH₂C(CH₃)₂CH₂OCH₃ | 3-CN | H | Racémate ; |
| isobutyle | CH₂CH₂OCH₃ | 3-CN | H | Racémate ; |
| cyclopentyle | CH₂CH₂OH | 3-CN | H | Racémate ; |
| cyclopentyle | CH₂CH₂OCH₃ | 3-CN | H | Racémate ; |
| cyclopentyle | CH₂CH₂OCH₂CH₃ | 3-CN | H | Racémate ; |
| cyclopentyle | CH₂CH₂OCH(CH₃)₂ | 3-CN | H | Raceémate ; |
| cyclopentyle | CH₂CH₂OCH(CH₃)₂ | 3-CN | H | Racémate ; |
| cyclopentyle | CH₂CH₂OC(CH₃)₃ | 3-CN | H | Racémate ; |
| cyclopentyle | CH₂C(CH₃)₂CH₂OCH₃ | 3-CN | H | Racémate ; |
| cyclohexyle | CH₂CH₂OCH₃ | 3-CN | H | Racémate ; |
| CH₂CH₂OCH₃ | CH₂CH₂OCH₃ | 3-CN | H | ; |
| cyclopentyle | CH₂CH₂OCH₃ | 2-CI | 5-CN | Racémate ; |
| cyclopentyle | méthyle | 2-CN | 3-Cl | Racémate ; |
| tertio-butyle | CH₂CH₂OCH₃ | 2-F | 6-F | Racémate ; |
| cyclopentyle | CH₂CH₂OCH(CH₃)₂ | 2-F | 3-F | Racémate ; |
| cyclopentyle | CH₂CH₂OH | 2-F | 3-F | Racémate ; |
| isopropyle | CH₂CH₂OH | 2-F | 3-F | Racémate ; |
| isopropyle | CH₂CH₂OCH(CH₃)₂ | 2-F | 3-F | Racémate ; |
| cycloheptyle | CH₂CH₂OCH₃ | 2-F | 5-F | Racémate ; |
| isopropyle | CH₂C(CH₃)₂OH | 2-F | 5-F | Racémate ; |
| méthyle | CH₂CH₂OCH₃ | 3-CN | H(-) | ; |
| méthyle | CH₂CH₂OCH₃ | 3-CN | H | (+) ; |
| isopropyle | CH₂CH₂OCH₃ | 2-Cl | 5-CN | (+) ; |
| isopropyle | CH₂CH₂OCH₃ | 2-Cl | 5-CN | (-) ; |
| CH₂CH₂OCH₃ | CH₂CH₂OCH₃ | 2-F | 3-CF₃ | ; |
| CH₂CH₂OCH₃ | CH₂CH₂OCH₃ | 2-Cl | 5-Cl | ; |
| CH₂CH₂OCH₃ | CH₂CH₂OCH₃ | 2-F | 5-F | ; |
| CH₂CH₂CH₂OCH₃ | CH₂CH₂CH₂OCH₃ | 2-Cl | 5-CI | ; |
| CH₂CH₂CH₂OCH₃ | CH₂CH₂CH₂OCH₃ | 2-F | 5-F | ; |
| CH₂C(CH₃)₂CH₂OCH₃ | CH₂C(CH₃)₂CH₂OCH₃ | 2-Cl | 5-Cl | ; |
| CH₂C(CH₃)₂CH₂OCH₃ | CH₂C(CH₃)₂CH₂OCH₃ | 2-F | 5-F | ; |
| méthyle | CH₂CH₂OCH₃ | 2-F | 3-F | Racémate ; |
| CH₂CH₂OCH₃ | CH₂CH₂OCH₃ | 2-F | 3-F | ; |
| CH₂CH₂OCH₃ | CH₂CH₂OCH₃ | 2-Cl | H | ; |
| isopropyle | CH₂CH₂OCH₃ | 2-CI | H | Racémate ; |
| | | | | ou |
| isopropyle | CH₂C(CH₃)₂CH₂OH | 2-Cl | H | Racémate |
ou leurs sels acceptables du point de vue physiologique.

9. Composés suivant la revendication 8, choisis dans le groupe des composés suivants :
(±)-(2-méthoxyéthyl)-4-(2-fluorophényl)-1,4-dihydro-2,6-diméthylpyridine-3,5-dicarboxylate d'isopropyle ;
(+)-(2-méthoxyéthyl)-4-(2-fluorophényl)-1,4-dihydro-2,6-diméthylpyridine-3,5-dicarboxylate d'isopropyle ;
(-)-(2-méthoxyéthyl)-4-(2-fluorophényl)-1,4-dihydro-2,6-diméthylpyridine-3,5-dicarboxylate d'isopropyle ;
(±)-(2-méthoxyéthyl)-4-(2,3-difluorophényl)-1,4-dihydro-2,6-diméthylpyridine-3,5-dicarboxylate d'isopropyle ;
(+)-(2-méthoxyéthyl)-4-(2,3-difluorophényl)-1,4-dihydro-2,6-diméthylpyridine-3,5-dicarboxylate d'isopropyle ;
(-)-(2-méthoxyéthyl)-4-(2,3-difluorophényl)-1,4-dihydro-2,6-diméthylpyridine-3,5-dicarboxylate d'isopropyle ;
(±)-(2-méthoxyéthyl)-4-(2,5-difluorophényl)-1,4-dihydro-2,6-diméthylpyridine-3,5-dicarboxylate d'isopropyle ;
(+)-(2-méthoxyéthyl)-4-(2,5-difluorophényl)-1,4-dihydro-2,6-diméthylpyridine-3,5-dicarboxylate d'isopropyle ;
(-)-(2-méthoxyéthyl)-4-(2,5-difluorophényl)-1,4-dihydro-2,6-diméthylpyridine-3,5-dicarboxylate d'isopropyle ;
(±)-(2-méthoxyéthyl)-4-(2,6-difluorophényl)-1,4-dihydro-2,6-diméthylpyridine-3,5-dicarboxylate d'isopropyle ;
(+)-(2-méthoxyéthyl)-4-(2,6-difluorophényl)-1,4-dihydro-2,6-diméthylpyridine-3,5-dicarboxylate d'isopropyle ;
(-)-(2-méthoxyéthyl)-4-(2,6-difluorophényl)-1,4-dihydro-2,6-diméthylpyridine-3,5-dicarboxylate d'isopropyle ;
(±)-(2-méthoxyéthyl)-4-(2,5-dichlorophényl)-1,4-dihydro-2,6-diméthylpyridine-3,5-dicarboxylate d'isopropyle ;
(+)-(2-méthoxyéthyl)-4-(2,5-dichlorophényl)-1,4-dihydro-2,6-diméthylpyridine-3,5-dicarboxylate d'isopropyle ;
(-)-(2-méthoxyéthyl)-4-(2,5-dichlorophényl)-1,4-dihydro-2,6-diméthylpyridine-3,5-dicarboxylate d'isopropyle ;
(±)-(2-méthoxyéthyl)-4-(2-chloro-6-fluorophényl)-1,4-dihydro-2,6-diméthylpyridine-3,5-dicarboxylate d'isopropyle ;
(+)-(2-méthoxyéthyl)-4-(2-chloro-6-fluorophényl)-1,4-dihydro-2,6-diméthylpyridine-3,5-dicarboxylate d'isopropyle ;
(-)-(2-méthoxyéthyl)-4-(2-chloro-6-fluorophényl)-1,4-dihydro-2,6-diméthylpyridine-3,5-dicarboxylate d'isopropyle ;
(±)-(2-méthoxyéthyl)-4-(2-fluoro-3-trifluorométhylphényl)-1,4-dihydro-2,6-diméthylpyridine-3,5-dicarboxylate d'isopropyle ;
(+)-(2-méthoxyéthyl)-4-(2-fluoro-3-trifluorométhylphényl)-1,4-dihydro-2,6-diméthylpyridine-3,5-dicarboxylate d'isopropyle ;
(-)-(2-méthoxyéthyl)-4-(2-fluoro-3-trifluorométhylphényl)-1,4-dihydro-2,6-diméthylpyridine-3,5-dicarboxylate d'isopropyle ;
(±)-(2-méthoxyéthyl)-4-(3-chloro-2-cyanophényl)-1,4-dihydro-2,6-diméthylpyridine-3,5-dicarboxylate d'isopropyle ;
(+)-(2-méthoxyéthyl)-4-(3-chloro-2-cyanophényl)-1,4-dihydro-2,6-diméthylpyridine-3,5-dicarboxylate d'isopropyle ;
(-)-(2-méthoxyéthyl)-4-(3-chloro-2-cyanophényl)-1,4-dihydro-2,6-diméthylpyridine-3,5-dicarboxylate d'isopropyle ;
(±)-(2-méthoxyéthyl)-4-(2-cyanophényl)-1,4-dihydro-2,6-diméthylpyridine-3,5-dicarboxylate d'isopropyle ;
(+)-(2-méthoxyéthyl)-4-(2-cyanophényl)-1,4-dihydro-2,6-diméthylpyridine-3,5-dicarboxylate d'isopropyle ;
(-)-(2-méthoxyéthyl)-4-(2-cyanophényl)-1,4-dihydro-2,6-diméthylpyridine-3,5-dicarboxylate d'isopropyle ;
(±)-(2-méthoxyéthyl)-4-(3-cyanophényl)-1,4-dihydro-2,6-diméthylpyridine-3,5-dicarboxylate de tertio-butyle ;
(+)-(2-méthoxyéthyl)-4-(3-cyanophényl)-1,4-dihydro-2,6-diméthylpyridine-3,5-dicarboxylate de tertio-butyle ;
(-)-(2-méthoxyéthyl)-4-(3-cyanophényl)-1,4-dihydro-2,6-diméthylpyridine-3,5-dicarboxylate de tertio-butyle ;
(±)-(2-méthoxyéthyl)-4-(3-cyanophényl)-1,4-dihydro-2,6-diméthylpyridine-3,5-dicarboxylate de cycloheptyle ;
(+)-(2-méthoxyéthyl)-4-(3-cyanophényl)-1,4-dihydro-2,6-diméthylpyridine-3,5-dicarboxylate de cycloheptyle ;
(-)-(2-méthoxyéthyl)-4-(3-cyanophényl)-1,4-dihydro-2,6-diméthylpyridine-3,5-dicarboxylate de cycloheptyle ;
(±)-(2-méthoxyéthyl)-4-(2-chloro-3-cyanophényl)-1,4-dihydro-2,6-diméthylpyridine-3,5-dicarboxylate de cyclopentyle ;
(+)-(2-méthoxyéthyl)-4-(2-chloro-3-cyanophényl)-1,4-dihydro-2,6-diméthylpyridine-3,5-dicarboxylate de cyclopentyle ;
(-)-(2-méthoxyéthyl)-4-(2-chloro-3-cyanophényl)-1,4-dihydro-2,6-diméthylpyridine-3,5-dicarboxylate de cyclopentyle ;
(±)-(2-méthoxyéthyl)-4-(2-fluoro-3-trifluorométhylphényl)-1,4-dihydro-2,6-diméthylpyridine-3,5-dicarboxylate de cyclopentyle ;
(+)-(2-méthoxyéthyl)-4-(2-fluoro-3-trifluorométhylphényl)-1,4-dihydro-2,6-diméthylpyridine-3,5-dicarboxylate de cyclopentyle ;
(-)-(2-méthoxyéthyl)-4-(2-fluoro-3-trifluorométhylphényl)-1,4-dihydro-2,6-diméthylpyridine-3,5-dicarboxylate de cyclopentyle ;
(±)-(2-méthoxyéthyl)-(méthyl)-4-(2-fluoro-3-trifluorométhylphényl)-1,4-dihydro-2,6-diméthylpyridine-3,5-dicarboxylate
(+)-(2-méthoxyéthyl)-(méthyl)-4-(2-fluoro-3-trifluorométhylphényl)-1,4-dihydro-2,6-diméthylpyridine-3,5-dicarboxylate
(-) - (2-méthoxyéthyl)-(méthyl)-4-(2-fluoro-3-trifluorométhylphényl)-1,4-dihydro-2,6-diméthylpyridine-3,5-dicarboxylate
(±)-(2-méthoxyéthyl)-4-(2-cyano-3-chlorophényl)-1,4-dihydro-2,6-diméthylpyridine-3,5-dicarboxylate de cyclopentyle ;
(+)-(2-méthoxyéthyl) -4- (2-cyano-3-chlorophényl) -1, 4-dihydro-2,6-diméthylpyridine-3,5-dicarboxylate de cyclopentyle ;
(-)-(2-méthoxyéthyl)-4-(2-cyano-3-chlorophényl)-1,4-dihydro-2,6-diméthylpyridine-3,5-dicarboxylate de cyclopentyle.
